# EUROPEAN PATENT SPECIFICATION

(11) **EP 0 904 410 B1**
(45) Date of publication and mention of the grant of the patent: **17.05.2006**
(21) Application number: 97925716.9
(22) Date of filing: 23.05.1997
(51) Int. Cl.: C12Q 1/68, C07H 21/04

(54) **IDENTIFICATION OF PYRAZINAMIDE-RESISTANT MYCOBACTERIA AND METHODS FOR TREATING MYCOBACTERIAL INFECTIONS**
ERKENNUNG VON PYRAZINAMID-RESISTENTEN MYCOBAKTERIEN UND VERFAHREN ZUR BEHANDLUNG VON MYCOBAKTERIEN-INFEKTIONEN
IDENTIFICATION DES MYCOBACTERIES RESISTANTES A LA PYRAZINAMIDE ET TRAITEMENT DES INFECTIONS MYCOBACTERIENNES

(30) Priority: 31.05.1996 US 655821
(43) Date of publication of application: 31.03.1999
(73) Proprietor: THE JOHNS HOPKINS UNIVERSITY, Baltimore, Maryland 21218-2685 (US)
(72) Inventor: ZHANG, Ying, Baltimore, MD 21234 (US); SCORPIO, Angelo, Columbia, MD 21045 (US)
(74) Representative: De Clercq, Ann G. Y.
(86) International application number: PCT/US1997/008770
(87) International publication number: WO 1997/045558

(56) References cited:
- EP-A- 0 528 306
- FROTHINGHAM R ET AL: "Cloning and expression of the pyrazinamidase gene." ABSTRACTS OF THE GENERAL MEETING OF THE AMERICAN SOCIETY FOR, vol. 96, 1996, page 114 XP009004159 96th General Meeting of the American Society for Microbiology;New Orleans, Louisiana, USA; May 19-23, 1996, 1996 ISSN: 1060-2011
- SPEIRS R J ET AL: "Activity of n-propyl pyrazinoate against pyrazinamide-resistant Mycobacterium tuberculosis: investigations into mechanism of action of and mechanism of resistance to pyrazinamide." ANTIMICROBIAL AGENTS AND CHEMOTHERAPY. UNITED STATES JUN 1995, vol. 39, no. 6, June 1995 (1995-06), pages 1269-1271, XP002228047 ISSN: 0066-4804
- KONNO K ET AL: "Pyrazinamide susceptibility and amidase activity of tubercle bacilli." THE AMERICAN REVIEW OF RESPIRATORY DISEASE. UNITED STATES MAR 1967, vol. 95, no. 3, March 1967 (1967-03), pages 461-469, XP009003947 ISSN: 0003-0805
- DEL PORTILLO P ET AL: "Multiprimer PCR system for differential identification of mycobacteria in clinical samples." JOURNAL OF CLINICAL MICROBIOLOGY. UNITED STATES FEB 1996, vol. 34, no. 2, February 1996 (1996-02), pages 324-328, XP002228048 ISSN: 0095-1137
- SCORPIO ANGELO ET AL: "Mutations in pncA, a gene encoding pyrazinamidase/nicotinamidase, cause resistance to the antituberculous drug pyrazinamide in tubercle bacillus." NATURE MEDICINE, vol. 2, no. 6, June 1996 (1996-06), pages 662-667, XP009003849 ISSN: 1078-8956
- NAT. MED., June 1996, Vol. 2, SCORPIO et al., "Mutations in PncA, a Gene Encoding Pyrazinamidase/Nicotinamidase, Cause Resistance to the Antituberculous Drug Pyrazinamide in Tubercle Bacillus", pages 662-667.

### SEQUENCE LISTING

(1) GENERAL INFORMATION:
   (i) APPLICANT: The Johns Hopkins University
   (ii) TITLE OF INVENTION: IDENTIFICATION OF PYRAZINAMIDE-RESISTANT MYCOBACTERIA AND METHODS FOR TREATING MYCOBACTERIAL INFECTIONS
   (iii) NUMBER OF SEQUENCES: 16
   (iv) CORRESPONDENCE ADDRESS:
      (A) ADDRESSEE: Fish & Richardson P.C.
      (B) STREET: 4225 Executive Square, Suite 1400
      (C) CITY: La Jolla
      (D) STATE: CA
      (E) COUNTRY: USA
      (F) ZIP: 92037
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patentln Release #1.0, Version #1.30
   (vii) PRIOR APPLICATION DATA:
      (A) APPLICATION NUMBER: US 08/655,821
      (B) FILING DATE: 31-MAY-1996
      (C) CLASSIFICATION:
   (viii) ATTORNEY/AGENT INFORMATION:
      (A) NAME: Haile, Lisa A.
      (B) REGISTRATION NUMBER: 38,347
      (C) REFERENCE/DOCKET NUMBER: 07662/003W01
   (ix) TELECOMMUNICATION INFORMATION:
      (A) TELEPHONE: 619/678-5070
      (B) TELEFAX: 619/678-5099
(2) INFORMATION FOR SEQ ID NO:1:
   (i) SEQUENCE CHARACTERISTICS:
      (A) LENGTH: 561 base pairs
      (B) TYPE: nucleic acid
      (C) STRANDEDNESS: single
      (D) TOPOLOGY: linear
   (ix) FEATURE:
      (A) NAME/KEY: CDS
      (B) LOCATION: 1..561
   (xi) SEQUENCE DESCRIPTION: SEQ ID NO:1:
   ATG CGG GCG TTG ATC ATC GTC GAC GTG CAG AAC GAC TTC TGC GAG GGT    48
   Met Arg Ala Leu Ile Ile Val Asp Val Gln Asn Asp Phe Cys Glu Gly
     1               5                  10                  15
   GGC TCG CTG GCG GTA ACC GGC GGC GCC GCG CTG GCC CGC GCC ATC AGC    96
   Gly Ser Leu Ala Val Thr Gly Gly Ala Ala Leu Ala Arg Ala Ile Ser
                20                  25                  30
   GAC TAC CTG GCC GAA GCG GCG GAC TAC CAT CAC GTC GTG GCA ACC AAG   144
   Asp Tyr Leu Ala Glu Ala Ala Asp Tyr His His Val Val Ala Thr Lys
            35                  40                  45
   GAC TTC CAC ATC GAC CCG GGT GAC CAC TTC TCC GGC ACA CCG GAC TAT   192
   Asp Phe His Ile Asp Pro Gly Asp His Phe Ser Gly Thr Pro Asp Tyr
        50                  55                  60
   TCC TCG TCG TGG CCA CCG CAT TGC GTC AGC GGT ACT CCC GGC GCG GAC   240
   Ser Ser Ser Trp Pro Pro His Cys Val Ser Gly Thr Pro Gly Ala Asp
    65                  70                  75                  80
   TTC CAT CCC AGT CTG GAC ACG TCG GCA ATC GAG GCG GTG TTC TAC AAG   288
   Phe His Pro Ser Leu Asp Thr Ser Ala Ile Glu Ala Val Phe Tyr Lys
                    85                  90                  95
   GGT GCC TAC ACC GGA GCG TAC AGC GGC TTC GAA GGA GTC GAC GAG AAC   336
   Gly Ala Tyr Thr Gly Ala Tyr Ser Gly Phe Glu Gly Val Asp Glu Asn
               100                 105                 110
   GGC ACG CCA CTG CTG AAT TGG CTG CGG CAA CGC GGC GTC GAT GAG GTC   384
   Gly Thr Pro Leu Leu Asn Trp Leu Arg Gln Arg Gly Val Asp Glu Val
           115                 120                 125
   GAT GTG GTC GGT ATT GCC ACC GAT CAT TGT GTG CGC CAG ACG GCC GAG   432
   Asp Val Val Gly Ile Ala Thr Asp His Cys Val Arg Gln Thr Ala Glu
       130                 135                 140
   GAC GCG GTA CGC AAT GGC TTG GCC ACC AGG GTG CTG GTG GAC CTG ACA   480
   Asp Ala Val Arg Asn Gly Leu Ala Thr Arg Val Leu Val Asp Leu Thr
   145                 150                 155                 160
   GCG GGT GTG TCG GCC CAT ACC ACC GTC GCC GCG CTG GAG GAG ATG CGC   528
   Ala Gly Val Ser Ala His Thr Thr Val Ala Ala Leu Glu Glu Met Arg
                   165                 170                 175
   ACC GCC AGC GTC GAG TTG GTT TGC AGC TCC TGA                       561
   Thr Ala Ser Val Glu Leu Val Cys Ser Ser  *
               180                 185
(2) INFORMATION FOR SEQ ID NO:2:
   (i) SEQUENCE CHARACTERISTICS:
      (A) LENGTH: 187 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Arg Ala Leu Ile Ile Val Asp Val Gln Asn Asp Phe Cys Glu Gly
   1 5 10 15
   Gly Ser Leu Ala Val Thr Gly Gly Ala Ala Leu Ala Arg Ala Ile Ser
   20 25 30
   Asp Tyr Leu Ala Glu Ala Ala Asp Tyr His His Val Val Ala Thr Lys
   35 40 45
   Asp Phe His Ile Asp Pro Gly Asp His Phe Ser Gly Thr Pro Asp Tyr
   50 55 60
   Ser Ser Ser Trp Pro Pro His Cys Val Ser Gly Thr Pro Gly Ala Asp
   65 70 75 80
   Phe His Pro Ser Leu Asp Thr Ser Ala Ile Glu Ala Val Phe Tyr Lys
   85 90 95
   Gly Ala Tyr Thr Gly Ala Tyr Ser Gly Phe Glu Gly Val Asp Glu Asn
   100 105 110
   Gly Thr Pro Leu Leu Asn Trp Leu Arg Gln Arg Gly Val Asp Glu Val
   115 120 125
   Asp Val Val Gly Ile Ala Thr Asp His Cys Val Arg Gln Thr Ala Glu
   130 135 140
   Asp Ala Val Arg Asn Gly Leu Ala Thr Arg Val Leu Val Asp Leu Thr
   145 150 155 160
   Ala Gly Val Ser Ala His Thr Thr Val Ala Ala Leu Glu Glu Met Arg
   165 170 175
   Thr Ala Ser Val Glu Leu Val Cys Ser Ser *
   180 185
(2) INFORMATION FOR SEQ ID NO:3:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:3:
   GATTGCCGAC GTGTCCAGAC 20
(2) INFORMATION FOR SEQ ID NO:4:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:4:
   ATCAGCGACT ACCTGGCCGA 20
(2) INFORMATION FOR SEQ ID NO:5:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:5:
   GCTGGTCATG TTCGCGATCG 20
(2) INFORMATION FOR SEQ ID NO:6:
   (i) SEQUENCE CHARACTERISTICS:
      (A) LENGTH: 20 base pairs
      (B) TYPE: nucleic acid
      (C) STRANDEDNESS: single
      (D) TOPOLOGY; linear
   (xi) SEQUENCE DESCRIPTION: SEQ ID NO:6:
   TCGGCCAGGT AGTCGCTGAT 20
(2) INFORMATION FOR SEQ ID NO:7:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:7:
   CGTCGACGTG CAGAACGACT 20
(2) INFORMATION FOR SEQ ID NO:8:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:8:
   GATTGCCGAC GTGTCCAGAC 20
(2) INFORMATION FOR SEQ ID NO:9:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:9:
   ACCGGACTAT TCCTCGTCGT 20
(2) INFORMATION FOR SEQ ID NO:10:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:10:
   GCGCACACAA TGATCGGTGG 20
(2) INFORMATION FOR SEQ ID NO:11:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:11:
   GCGGCTTCGA AGGAGTCGAC 20
(2) INFORMATION FOR SEQ ID NO:12:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:12:
   GCTTTGCGGC GAGCGCTCCA 20
(2) INFORMATION FOR SEQ ID NO:13:
   (i) SEQUENCE CHARACTERISTICS:
      (A) LENGTH: 9 amino acids
      (B) TYPE: amino acid
      (C) STRANDEDNESS: not relevant
      (D) TOPOLOGY: linear
   (ii) MOLECULE TYPE: protein
   (xi) SEQUENCE DESCRIPTION: SEQ ID NO:13:
   Val Asp Leu Gln Asn Asp Phe Cys Ala
   1               5
(2) INFORMATION FOR SEQ ID NO:14:
   (i) SEQUENCE CHARACTERISTICS:
      (A) LENGTH: 11 amino acids
      (B) TYPE: amino acid
      (C) STRANDEDNESS: not relevant
      (D) TOPOLOGY: linear
   (ii) MOLECULE TYPE: protein
   (xi) SEQUENCE DESCRIPTION: SEQ ID NO:14:
   Gly Tyr Lys Val Asn Val Ile Thr Asp Gly Cys
   1               5                   10
(2) INFORMATION FOR SEQ ID NO:15:
   (i) SEQUENCE CHARACTERISTICS:
      (A) LENGTH: 186 amino acids
      (B) TYPE: amino acid
      (C) STRANDEDNESS: not relevant
      (D) TOPOLOGY: linear
   (ii) MOLECULE TYPE: protein
   (xi) SEQUENCE DESCRIPTION: SEQ ID NO:15:
   Met Arg Ala Leu Ile Ile Val Asp Val Gln Asn Asp Phe Cys Glu Gly
   1               5                   10                  15
   Gly Ser Leu Ala Val Thr Gly Gly Ala Ala Leu Ala Arg Ala Ile Ser
               20                  25                  30
   Asp Tyr Leu Ala Glu Ala Ala Asp Tyr His His Val Val Ala Thr Lys
           35                  40                  45
   Asp Phe His Ile Asp Pro Gly Asp His Phe Ser Gly Thr Pro Asp Tyr
       50                  55                  60
   Ser Ser Ser Trp Pro Pro His Cys Val Ser Gly Thr Pro Gly Ala Asp
   65                  70                  75                  80
   Phe His Pro Ser Leu Asp Thr Ser Ala Ile Glu Ala Val Phe Tyr Lys
                   85                  90                  95
   Gly Ala Tyr Thr Gly Ala Tyr Ser Gly Phe Glu Gly Val Asp Glu Asn
               100                 105                 110
   Gly Thr Pro Leu Leu Asn Trp Leu Arg Gln Arg Gly Val Asp Glu Val
           115                 120                 125
   Asp Val Val Gly Ile Ala Thr Asp His Cys Val Arg Gln Thr Ala Glu
       130                 135                 140
   Asp Ala Val Arg Asn Gly Leu Ala Thr Arg Val Leu Val Asp Leu Thr
   145                 150                 155                 160
   Ala Gly Val Ser Ala Asp Ile Thr Val Ala Ala Leu Glu Glu Met Arg
                   165                 170                 175
   Thr Ala Ser Val Glu Leu Val Cys Ser Ser
               180                 185
(2) INFORMATION FOR SEQ ID NO:16:
   (i) SEQUENCE CHARACTERISTICS:
      (A) LENGTH: 213 amino acids
      (B) TYPE: amino acid
      (C) STRANDEDNESS: not relevant
      (D) TOPOLOGY: linear
   (ii) MOLECULE TYPE: protein
   (xi) SEQUENCE DESCRIPTION: SEQ ID NO:16:
   Met Pro Pro Arg Ala Leu Leu Leu Val Asp Leu Gln Asn Asp Phe Cys
   1               5                   10                  15
   Ala Gly Gly Ala Leu Ala Val Pro Glu Gly Asp Ser Thr Val Asp Val
               20                  25                  30
   Ala Asn Arg Leu Ile Asp Trp Cys Gln Ser Arg Gly Glu Ala Val Ile
           35                  40                  45
   Ala Ser Gln Asp Trp His Pro Ala Asn His Gly Ser Phe Ala Ser Gln
       50                  55                  60
   His Gly Val Glu Pro Tyr Thr Pro Gly Gln Leu Asp Gly Leu Pro Gln
   65                  70                  75                  80
   Thr Phe Trp Pro Asp His Cys Val Gln Asn Ser Glu Gly Ala Gln Leu
                   85                  90                  95
   His Pro Leu Leu His Gln Lys Ala Ile Ala Ala Val Phe His Lys Gly
   100 105 110
   Glu Asn Pro Leu Val Asp Ser Tyr Ser Ala Phe Phe Asp Asn Gly Arg
   115 120 125
   Arg Gln Lys Thr Ser Leu Asp Asp Trp Leu Arg Asp His Glu Ile Asp
   130 135 140
   Glu Leu Ile Val Met Gly Leu Ala Thr Asp Tyr Cys Val Lys Phe Thr
   145 150 155 160
   Val Leu Asp Ala Leu Gln Leu Gly Tyr Lys Val Asn Val Ile Thr Asp
   165 170 175
   Gly Cys Arg Gly Val Asn Ile Gln Pro Gln Asp Ser Ala His Ala Phe
   180 185 190
   Met Glu Met Ser Ala Ala Gly Ala Thr Leu Tyr Thr Leu Ala Asp Trp
   195 200 205
   Glu Glu Thr Gln Gly
   210

### SEQUENCE LISTING

(1) GENERAL INFORMATION:
   (i) APPLICANT:
      (A) NAME: The Johns Hopkins University
      (B) STREET: 34th and Charles Street
      (C) CITY: Baltimore, Maryland
      (D) COUNTRY: United States of America
      (F) POSTAL CODE (ZIP): MD 21218
   (ii) TITLE OF INVENTION: IDENTIFICATION OF PYRAZINAMIDE-RESISTANT MYCOBACTERIA AND METHODS FOR TREATING MYCOBACTERIAL INFECTIONS
   (iii) NUMBER OF SEQUENCES: 16
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
(2) INFORMATION FOR SEQ ID NO:1:
   (i) SEQUENCE CHARACTERISTICS:
      (A) LENGTH: 561 base pairs
      (B) TYPE: nucleic acid
      (C) STRANDEDNESS: single
      (D) TOPOLOGY: linear
   (ix) FEATURE:
      (A) NAME/KEY: CDS
      (B) LOCATION: 1..561
   (xi) SEQUENCE DESCRIPTION: SEQ ID NO:1:
   ATG CGG GCG TTG ATC ATC GTC GAC GTG CAG AAC GAC TTC TGC GAG GGT      48
   Met Arg Ala Leu Ile Ile Val Asp Val Gln Asn Asp Phe Cys Glu Gly
     1               5                  10                  15
   GGC TCG CTG GCG GTA ACC GGC GGC GCC GCG CTG GCC CGC GCC ATC AGC      96
   Gly Ser Leu Ala Val Thr Gly Gly Ala Ala Leu Ala Arg Ala Ile Ser
                20                  25                  30
   GAC TAC CTG GCC GAA GCG GCG GAC TAC CAT CAC GTC GTG GCA ACC AAG     144
   Asp Tyr Leu Ala Glu Ala Ala Asp Tyr His His Val Val Ala Thr Lys
            35                  40                  45
   GAC TTC CAC ATC GAC CCG GGT GAC CAC TTC TCC GGC ACA CCG GAC TAT     192
   Asp Phe His Ile Asp Pro Gly Asp His Phe Ser Gly Thr Pro Asp Tyr
        50                  55                  60
   TCC TCG TCG TGG CCA CCG CAT TGC GTC AGC GGT ACT CCC GGC GCG GAC     240
   Ser Ser Ser Trp Pro Pro His Cys Val Ser Gly Thr Pro Gly Ala Asp
    65                  70                  75                  80
   TTC CAT CCC AGT CTG GAC ACG TCG GCA ATC GAG GCG GTG TTC TAC AAG     288
   Phe His Pro Ser Leu Asp Thr Ser Ala Ile Glu Ala Val Phe Tyr Lys
                    85                  90                  95
   GGT GCC TAC ACC GGA GCG TAC AGC GGC TTC GAA GGA GTC GAC GAG AAC     336
   Gly Ala Tyr Thr Gly Ala Tyr Ser Gly Phe Glu Gly Val Asp Glu Asn
               100                 105                 110
   GGC ACG CCA CTG CTG AAT TGG CTG CGG CAA CGC GGC GTC GAT GAG GTC     384
   Gly Thr Pro Leu Leu Asn Trp Leu Arg Gln Arg Gly Val Asp Glu Val
           115                 120                 125
   GAT GTG GTC GGT ATT GCC ACC GAT CAT TGT GTG CGC CAG ACG GCC GAG     432
   Asp Val Val Gly Ile Ala Thr Asp His Cys Val Arg Gln Thr Ala Glu
       130                 135                 140
   GAC GCG GTA CGC AAT GGC TTG GCC ACC AGG GTG CTG GTG GAC CTG ACA     480
   Asp Ala Val Arg Asn Gly Leu Ala Thr Arg Val Leu Val Asp Leu Thr
   145                 150                 155                 160
   GCG GGT GTG TCG GCC CAT ACC ACC GTC GCC GCG CTG GAG GAG ATG CGC     528
   Ala Gly Val Ser Ala His Thr Thr Val Ala Ala Leu Glu Glu Met Arg
                   165                 170                 175
   ACC GCC AGC GTC GAG TTG GTT TGC AGC TCC TGA                         561
   Thr Ala Ser Val Glu Leu Val Cys Ser Ser  *
               180                 185
(2) INFORMATION FOR SEQ ID NO:2:
   (i) SEQUENCE CHARACTERISTICS:
      (A) LENGTH: 187 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Arg Ala Leu Ile Ile Val Asp Val Gln Asn Asp Phe Cys Glu Gly
    1               5                  10                  15
   Gly Ser Leu Ala Val Thr Gly Gly Ala Ala Leu Ala Arg Ala Ile Ser
               20                  25                  30
   Asp Tyr Leu Ala Glu Ala Ala Asp Tyr His His Val Val Ala Thr Lys
           35                  40                  45
   Asp Phe His Ile Asp Pro Gly Asp His Phe Ser Gly Thr Pro Asp Tyr
       50                  55                  60
   Ser Ser Ser Trp Pro Pro His Cys Val Ser Gly Thr Pro Gly Ala Asp
    65                  70                  75                  80
   Phe His Pro Ser Leu Asp Thr Ser Ala Ile Glu Ala Val Phe Tyr Lys
                   85                  90                  95
   Gly Ala Tyr Thr Gly Ala Tyr Ser Gly Phe Glu Gly Val Asp Glu Asn
               100                 105                 110
   Gly Thr Pro Leu Leu Asn Trp Leu Arg Gln Arg Gly Val Asp Glu Val
           115                 120                 125
   Asp Val Val Gly Ile Ala Thr Asp His Cys Val Arg Gln Thr Ala Glu
       130                 135                 140
   Asp Ala Val Arg Asn Gly Leu Ala Thr Arg Val Leu Val Asp Leu Thr
   145                 150                 155                 160
   Ala Gly Val Ser Ala His Thr Thr Val Ala Ala Leu Glu Glu Met Arg
                   165                 170                 175
   Thr Ala Ser Val Glu Leu Val Cys Ser Ser  *
               180                 185
(2) INFORMATION FOR SEQ ID NO:3:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:3:
   GATTGCCGAC GTGTCCAGAC 20
(2) INFORMATION FOR SEQ ID NO:4:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:4:
   ATCAGCGACT ACCTGGCCGA 20
(2) INFORMATION FOR SEQ ID NO:5:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:5:
   GCTGGTCATG TTCGCGATCG 20
(2) INFORMATION FOR SEQ ID NO:6:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:6:
   TCGGCCAGGT AGTCGCTGAT 20
(2) INFORMATION FOR SEQ ID NO:7:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:7:
   CGTCGACGTG CAGAACGACT 20
(2) INFORMATION FOR SEQ ID NO:8:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
      (xi) SEQUENCE DESCRIPTION: SEQ ID NO:8:
   GATTGCCGAC GTGTCCAGAC 20
(2) INFORMATION FOR SEQ ID NO:9:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:9:
   ACCGGACTAT TCCTCGTCGT 20
(2) INFORMATION FOR SEQ ID NO:10:
   (i) SEQUENCE CHARACTERISTICS:
      (A) LENGTH: 20 base pairs
      (B) TYPE: nucleic acid
      (C) STR.ANDEDNESS: single
      (D) TOPOLOGY: linear
   (xi) SEQUENCE DESCRIPTION: SEQ ID NO:10:
   GCGCACACAA TGATCGGTGG 20
(2) INFORMATION FOR SEQ ID NO:11:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:11:
   GCGGCTTCGA AGGAGTCGAC 20
(2) INFORMATION FOR SEQ ID NO:12:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:12:
   GCTTTGCGGC GAGCGCTCCA 20
(2) INFORMATION FOR SEQ ID NO:13:
   (i) SEQUENCE CHARACTERISTICS:
      (A) LENGTH: 9 amino acids
      (B) TYPE: amino acid
      (C) STRANDEDNESS: not relevant
      (D) TOPOLOGY: linear
   (ii) MOLECULE TYPE: protein
   (xi) SEQUENCE DESCRIPTION: SEQ ID NO:13:
   Val Asp Leu Gln Asn Asp Phe Cys Ala
   1 5
(2) INFORMATION FOR SEQ ID NO:14:
   (i) SEQUENCE CHARACTERISTICS:
      (A) LENGTH: 11 amino acids
      (B) TYPE: amino acid
      (C) STRANDEDNESS: not relevant
      (D) TOPOLOGY: linear
   (ii) MOLECULE TYPE: protein
   (xi) SEQUENCE DESCRIPTION: SEQ ID NO:14:
   Gly Tyr Lys Val Asn Val Ile Thr Asp Gly Cys
   1 5 10
(2) INFORMATION FOR SEQ ID NO:15:
   (i) SEQUENCE CHARACTERISTICS:
      (A) LENGTH: 186 amino acids
      (B) TYPE: amino acid
      (C) STRANDEDNESS: not relevant
      (D) TOPOLOGY: linear
   (ii) MOLECULE TYPE: protein
   (xi) SEQUENCE DESCRIPTION: SEQ ID NO:15:
   Met Arg Ala Leu Ile Ile Val Asp Val Gln Asn Asp Phe Cys Glu Gly
   1               5                   10                  15
   Gly Ser Leu Ala Val Thr Gly Gly Ala Ala Leu Ala Arg Ala Ile Ser
               20                  25                  30
   Asp Tyr Leu Ala Glu Ala Ala Asp Tyr His His Val Val Ala Thr Lys
           35                  40                  45
   Asp Phe His Ile Asp Pro Gly Asp His Phe Ser Gly Thr Pro Asp Tyr
       50                  55                  60
   Ser Ser Ser Trp Pro Pro His Cys Val Ser Gly Thr Pro Gly Ala Asp
   65                  70                  75                  80
   Phe His Pro Ser Leu Asp Thr Ser Ala Ile Glu Ala Val Phe Tyr Lys
                   85                  90                  95
   Gly Ala Tyr Thr Gly Ala Tyr Ser Gly Phe Glu Gly Val Asp Glu Asn
               100                 105                 110
   Gly Thr Pro Leu Leu Asn Trp Leu Arg Gln Arg Gly Val Asp Glu Val
           115                 120                 125
   Asp Val Val Gly Ile Ala Thr Asp His Cys Val Arg Gln Thr Ala Glu
       130                 135                 140
   Asp Ala Val Arg Asn Gly Leu Ala Thr Arg Val Leu Val Asp Leu Thr
   145                 150                 155                 160
   Ala Gly Val Ser Ala Asp Ile Thr Val Ala Ala Leu Glu Glu Met Arg
                   165                 170                 175
   Thr Ala Ser Val Glu Leu Val Cys Ser Ser
               180                 185
(2) INFORMATION FOR SEQ ID NO:16:
   (i) SEQUENCE CHARACTERISTICS:
      (A) LENGTH: 213 amino acids
      (B) TYPE: amino acid
      (C) STRANDEDNESS: not relevant
      (D) TOPOLOGY: linear
   (ii) MOLECULE TYPE: protein
   (xi) SEQUENCE DESCRIPTION: SEQ ID NO:16:
   Met Pro Pro Arg Ala Leu Leu Leu Val Asp Leu Gln Asn Asp Phe Cys
   1               5                   10                  15
   Ala Gly Gly Ala Leu Ala Val Pro Glu Gly Asp Ser Thr Val Asp Val
               20                  25                  30
   Ala Asn Arg Leu Ile Asp Trp Cys Gln Ser Arg Gly Glu Ala Val Ile
           35                  40                  45
   Ala Ser Gln Asp Trp His Pro Ala Asn His Gly Ser Phe Ala Ser Gln
       50                  55                  60
   His Gly Val Glu Pro Tyr Thr Pro Gly Gln Leu Asp Gly Leu Pro Gln
   65                  70                  75                  80
   Thr Phe Trp Pro Asp His Cys Val Gln Asn Ser Glu Gly Ala Gln Leu
                   85                  90                  95
   His Pro Leu Leu His Gln Lys Ala Ile Ala Ala Val Phe His Lys Gly
               100                 105                 110
   Glu Asn Pro Leu Val Asp Ser Tyr Ser Ala Phe Phe Asp Asn Gly Arg
           115                 120                 125
   Arg Gln Lys Thr Ser Leu Asp Asp Trp Leu Arg Asp His Glu Ile Asp
       130                 135                 140
   Glu Leu Ile Val Met Gly Leu Ala Thr Asp Tyr Cys Val Lys Phe Thr
   145                 150                 155                 160
   Val Leu Asp Ala Leu Gln Leu Gly Tyr Lys Val Asn Val Ile Thr Asp
                   165                 170                 175
   Gly Cys Arg Gly Val Asn Ile Gln Pro Gln Asp Ser Ala His Ala Phe
               180                 185                 190
   Met Glu Met Ser Ala Ala Gly Ala Thr Leu Tyr Thr Leu Ala Asp Trp
           195                 200                 205
   Glu Glu Thr Gln Gly
       210

## Description

### Field of the Invention

This invention relates to methods and nucleic acids for identifying pyrazinamide-resistant mycobacteria, including *M. bovis*, and for treating infections with pyrazinamide-resistant mycobacteria.

### Background of the Invention

Tuberculosis in humans and other mammals is caused by members of the *M. tuberculosis* complex, which includes *M. tuberculosis, M. bovis, M. africanum, and M. microti. M. bovis* and *M. tuberculosis* are closely related, and share more than 99% sequence identity. Although *M. bovis* is the primary cause of tuberculosis in cattle, it is transmissible to humans, where it can cause tuberculosis.

Conventional methods for treating tuberculosis include administration of drugs such as pyrazinamide, isoniazid (INH), rifampicin, streptomycin, and ethambutol. Pyrazinamide (PZA) is particularly useful because it kills certain semi-dormant bacteria that are not affected by other anti-tuberculosis drugs (McCune et al., 1956, J. Exp. Med. 104: 763-802; Heifets et al., 1992, Am. Rev. Respir. Dis. 145:1223-1225). Thus, inclusion of PZA with INH and rifampicin considerably shortens the typical treatment period from 12-18 months to 6 months, and the three drugs together form the basis of the standard short course chemotherapy iMcC`une et al., J. Exp. Med. 104, 763-802 (1956); Mitchison, D.S., Tubercle 66, 219-225 (1985)).

Drug-resistant mycobacteria pose a considerable threat to the control of tuberculosis. Indeed, multidrug-resistant (MDR) strains of *M. tuberculosis* have caused several fatal outbreaks in both HIV-positive and HIV-negative individuals (Centers for Disease Control, Florida and New York, 1988-1991, Mortal, Morbid, Weekly Rep. 40, 585-591 (1991); Bloom, et al., Science 257, 1055-1064 (1992)). Strains of *M. bovis* are naturally resistant to PZA, and several strains of *M. tuberculosis* are known to have acquired PZA resistance. Examples of acquired PZA-resistant strains include PZA-R (ATCC 35828) and the clinical isolate M36470, M3S169, F36946, and Vertullo. Because PZA is a commonly used anti-tuberculosis drug, the existence of PZA-resistant strains has complicated efforts to combat tuberculosis infections. Thus, it is important to determine whether the infecting mycobacterium is *M. bovis* or a PZA-susceptible variant of *M. tuberculosis* in order to know whether PZA-based therapy is feasible. Yet, prior to the work described below, the molecular basis of pyrazinamide (PZA) resistance remained unknown. The traditional methods for identifying PZA-resistant mycobacteria, such as *M. bovis*, typically involve time-consuming biochemical assays, which require 2-8 weeks to perform.

### Summary of the Invention

The present invention is based on the discovery of the molecular basis for PZA-resistance in mycobacteria such as *M. bovis.* PZA resistance is conferred by an alteration(s) in the *pnc*A gene of both naturally PZA-resistant mycobacteria and acquired PZA-resistant strains of mycobacteria. The altered *pncA* genes of PZA-resistant mycobacteria fail to encode functional pyrazinamidase polypeptides.

Accordingly, the invention provides methods, primers, probes, and kits useful for identifying mycobacteria that are resistant to PZA due to alterations in the *pncA* gene. Also included within the invention are nucleic acids encoding altered PZase polypeptides that confer PZA resistance and wild-type *M. tuberculosis* PZase polypeptide. A substantially purified wild-type *M. tuberculosis* PZase polypeptide and substantially purified altered PZase polypeptides that confer PZA resistance, and antibodies that specifically bind such polypeptides, also are included in the invention. The identification of PZA-resistant mycobacteria is clinically useful for directing the treatment of tuberculosis patients. In addition, conventional biochemical methods for identifying PZA-resistant bacteria can produce initially false results. By providing molecular techniques that have a relatively high degree of accuracy, the invention facilities the rapid detection of PZA-resistant bacteria, and augments the design of new anti-tuberculosis drugs (e.g., PZA derivatives), which requires accurate identification of the tested mycobacteria.

Derived from the discovery that *M. bovis* naturally has an altered *pncA* gene, the invention provides methods, primers, probes, substantially purified polypeptides, and isolated antibodies for distinguishing between *M. bovis* and *M. tuberculosis.* These methods, nucleic acids, polypeptides, and antibodies are particularly useful as epidemiological tools to monitor the spread of bovine tuberculosis to humans in geographic areas that face tuberculosis outbreaks. For commercial purposes, the primers and probes of the invention can be packaged into a kit.

The invention also provides methods for treating a mammal that is infected with PZA-resistant mycobacteria. The methods involve (i) introducing into the mycobacteria a functional *pncA* gene, and (ii) administering PZA to the mammal, thereby killing the mycobacteria.

### Brief Description of the Drawings

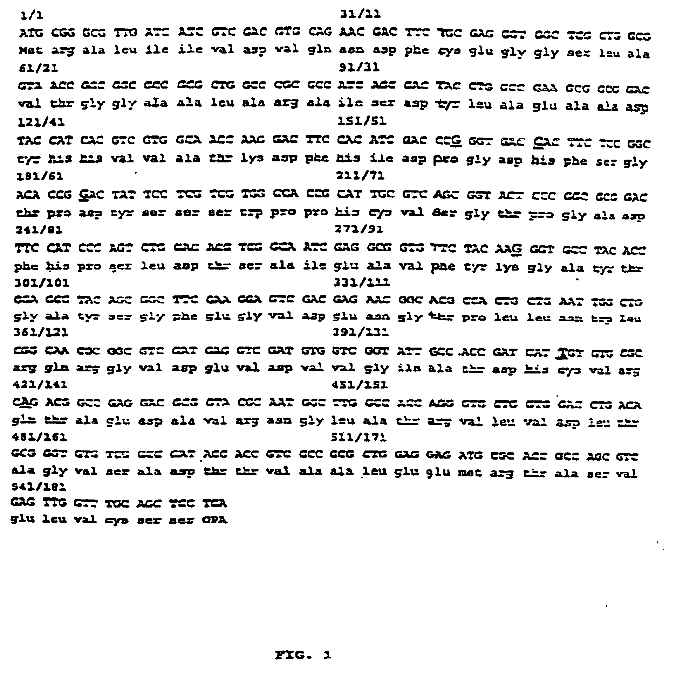

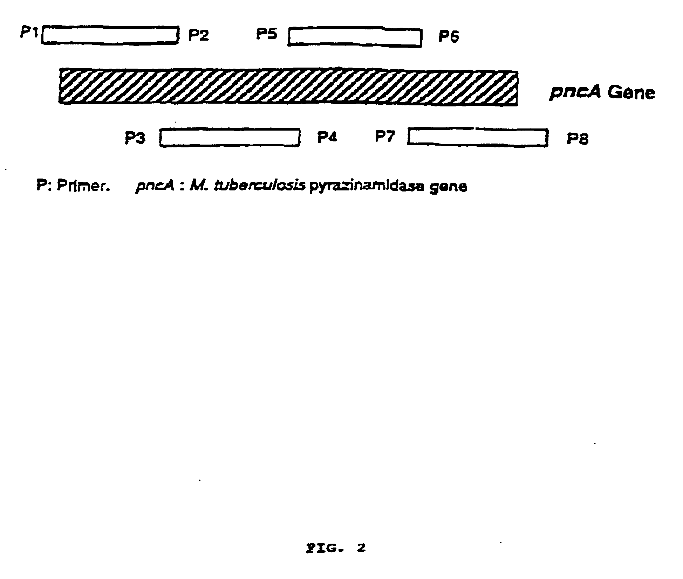

Fig. 1 is a listing of the DNA and amino acid sequences of the wild-type *pncA* gene of *M. tuberculosis.* The nucleotides that are altered in the altered *pncA* genes discussed below are indicated by bold-face type and underscoring. The complete sequence of the *M. tuberculosis pncA* gene was obtained from the 3.2 kb DNA fragment (Erdman strain) using primers designed from the sequence of the partial pncA-containing 500 bp PCR product.

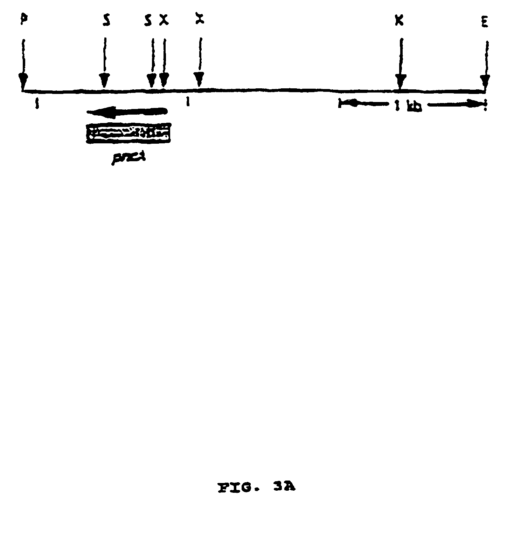

Fig. 2 is a schematic representation of the *pncA* gene and the portions of the gene that are amplified by particular pairs of primers (P1-P8).

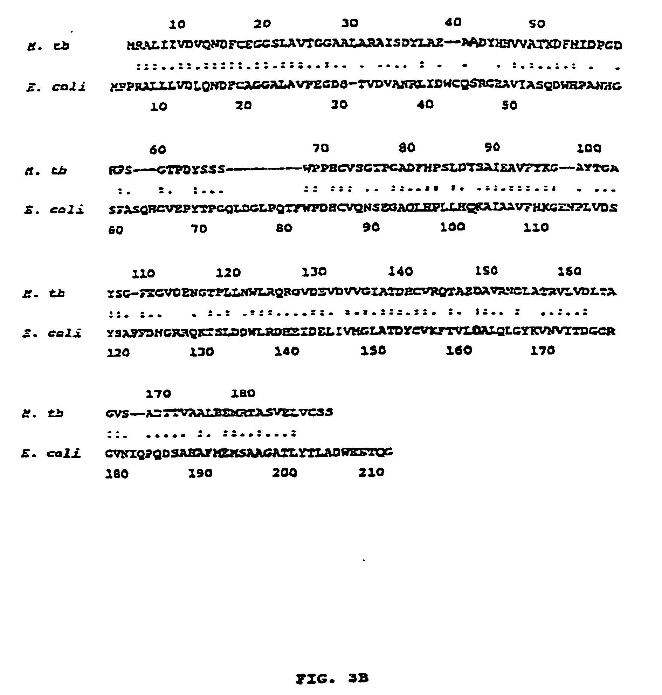

Fig. 3A is a restriction map of a 3.2 kb *Eco*RI-*Pst*I fragment containing the *pncA*. The 3.2 kb fragment was derived from cosmid DNA isolated using a 500 bp *pncA*-containing PCR product as a probe. The bold arrow indicates the direction of transcription for the *pnc*A gene. Restriction endonuclease recognition sites are abbreviated as: E, *Eco*RI; K, *Kpn*I; P, *Pst*I; S, *Sma*I; and X, *Xmn*I.
Fig. 3B is a pair of nucleotide sequences showing the limited homology between the genes encoding *M. tuberculosis* PZase and *E. coli* nicotinamidase.

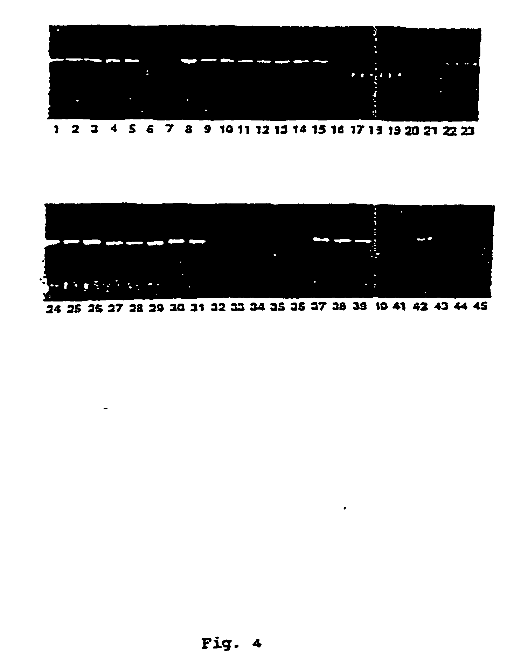

Fig. 4 is a pair of photographs showing that the mobility of DNA corresponding to *M. bovis* differs from the mobility of DNA corresponding to *M. tuberculosis* when analyzed by PCR-SSCP. *M. bovis:* lanes 1-5, 8-15, 20-23, 24-31, 36-39, and 42. *M. tuberculosis:* lanes 6-7, 16-19, 32-35, 40-41, and 43-45.

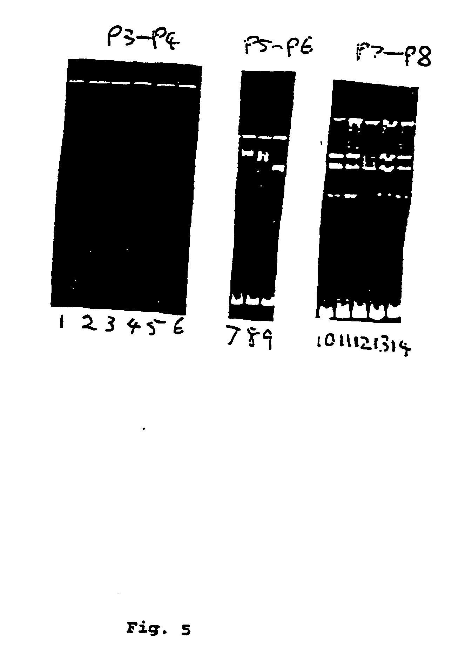

Fig. 5 is a set of photographs showing that the mobility of DNA corresponding to PZA-resistant *M. tuberculosis* differs from the mobility of DNA corresponding to wild-type (PZA-sensitive) *M. tuberculosis* when analyzed by PCR-SSCP. To amplify the DNA electrophoresed in lanes 1-6, primers P3 (SEQ ID NO: 7) and P4 (SEQ ID NO: 8) were used. To amplify the DNA electrophoresed in lanes 7-9, primers P5 (SEQ ID NO: 9) and P6 (SEQ ID NO: 10) were used. Primers P7 (SEQ ID NO: 11) and P8 (SEQ ID NO: 12) were used to amplify the DNA that is electrophoresed in lanes 10-14. DNA amplified from PZA-resistant *M. tuberculosis* strains is electrophoresed in lanes 1-4, 5, 8, 9, and 12. As a control, DNA amplified from a wild-type (PZA-sensitive) *M. tuberculosis* strain is electrophoresed in lanes 5, 7, 10, 11, 13, and 14. The mycobacterial strains were: *M*. *bovis* (ATCC 19210) in lane 1; BCG Tokyo in lane 2 (provided by P. Converse, Johns Hopkins University); PZA-resistant clinical isolates of *M. tuberculosis* from Brazil in lanes 3-6 (provided by S. Morris; FDA); PZA-sensitive *M**.** tuberculosis* strain 12646 in lane 7; PZA-resistant *M. tuberculosis* strain derived from type strain H37Rv (ATCC 35828) in lane 8; BCG Tice in lane 9 (provided by P. Converse); PZA-sensitive *M. tuberculosis* Erdman strain in lanes 10, 11, 13, and 14 (H37728, 12646, and M40023); and PZA-resistant *M. tuberculosis* strain M3169 in lane 12.

### A Detailed Description of the Invention

Differentiation Between M. *bovis* and *M. tuberculosis:* In one aspect, this invention provides a method for differentiating between *M. tuberculosis* and *M. bovis* in a sample. The method involves detecting in the mycobacteria of the sample an altered *pncA* gene that encodes an aspartic acid residue, rather than a histidine residue, at amino acid position 57 of the PZase polypeptide encoded by the altered *pncA* gene. All strains of *M. bovis* that have been examined to date have this alteration, whereas this alteration has not been found in any strain of *M. tuberculosis*. Thus, an aspartic acid residue at this position is indicative of *M. bovis,* rather than *M. tuberculosis*. The aspartic acid residue at position 57 can be encoded by a change of C to G at nucleotide 169 of the altered *pncA* gene. Other alterations in nucleotide sequences can also result in an aspartic acid residue at position 57. For example, because of the degeneracy of the genetic code, a change of C to G at nucleotide 169 combined with a change of C to t1 at nucleotide 171 also will encode an aspartic acid residue at amino acid position 57.

As used herein, the term "altered" refers to any *pncA* nucleic acid (e.g., a gene) or PZase polypeptide that differs in nucleotide or amino acid sequence from the wild-type *pncA* gene of *M. tuberculosis* (or degenerate variants thereof) or the wild-type PZase polypeptide of *M. tuberculosis*. Thus, the *M. bovis pncA* gene is considered an "altered" *pncA* gene, even though this gene naturally confers PZA resistance. The "wild-type" *M. tuberculosis pncA* gene and the wild-type PZase polypeptide are listed in Fig. 1 (SEQ ID NO: 1 and SEQ ID NO: 2). While the nucleic acid sequences of "degenerate variants" of the *pncA* gene differ from the illustrated wild-type *pncA* sequence, the degenerate variants nonetheless encode a wild-type PZase polypeptide because most of the 20 natural amino acids are each specified by more than one codon. Thus, a "degenerate variant" of a nucleotide sequence is a nucleotide sequence that encodes the same amino acid sequence as a given nucleotide sequence, but in which at least one codon in the nucleotide sequence is different because two or more different codons can encode the same amino acid. All degenerate nucleotide sequences are included in the invention, as long as the amino acid sequence of the *pncA* polypeptide encoded by the nucleotide sequence is functionally unchanged. In addition, the invention includes an isolated nucleic acid encoding a polypeptide having the biological activity of an amino acid sequence of SEQ ID NO:2 and having at least one epitope for an antibody immunoreactive with PZase polypeptide.

An isolated nucleic acid encoding an *M. cuberculosis* PZase polypeptide is included within the invention, and useful for production of the primers and probes described herein. The term "isolated" as used herein includes polynucleotides or polypeptides substantially free of other nucleic acids, proteins, lipids, carbohydrates, or other materials with which it is naturally associated. The isolated nucleic acids of the invention encoding *M. tuberculosis* PZase polypeptide include nucleic acids that encode "conservative variations" of the PZase polypeptide. A conservative variation as used herein denotes the replacement of an amino acid residue by another, biologically similar, residue. Examples of conservative variations include the substitution of one hydrophobic residue, such as isoleucine, valine, leucine, or methionine, for another, or the substitution of one polar residue for another, such as the substitution of arginine for lysine, glutamic acid for aspartic acid, or glutamine for asparagine, and the like. The term "conservative variation" also includes the use of a substituted amino acid in place of an unsubstituted parent amino acid, provided that antibodies raised to the substituted polypeptide also immunoreact with the unsubstituted polypeptide. Also include are isolated nucleic acids that encode a portion or fragment of the PZase polypeptide, as long as the PZase polypeptide retains a biological activity of the full length PZase polypeptide, such as the ability to convert PZA to pyrazinoic acid (Konno et al., Am. Rev. Respir. Dis. 95, 461-469 (1967)). Such nucleic acids include naturally-occurring, synthetic, and intentionally manipulated polynucleotides. For example, portions of the mRNA sequence may be altered due to alternate promoters for RNA transcription. As another example, *pncA* nucleic acids may be subjected to site-directed mutagenesis. Antisense sequences for *pncA* also are included.

Any of the various art-known methods for detecting point mutations can be used to detect the altered *M. bovis pncA* gene. Now that the alteration in the *M. bovis* sequence has been identified and appreciated, a person of ordinary skill in molecular biology can readily identify *M. bovis* using any conventional method for detecting point mutations. The term "detecting" as used herein encompasses any means for determining the presence of a given gene or polypeptide in a sample. For example, an altered *pncA* gene can be detected by methods such as, but not limited to, polymerase chain reaction single-strand conformation polymorphism (PCR-SSCP (Orita et al., 1989, Genomics 5:874-879)), single-strand conformation polymorphism (SSCP (Orita et al., 1989, Proc. Natl. Acad. Sci. 86:2766-2770)), DNA sequencing, DNA hybridization (e.g., Southern blotting, dot/slot blotting, colony hybridization), denaturing gradient gel electrophoresis (Myers et al., 1985, Nature, 313:495-498), ligase-mediated gene detection (Landegren et al., 1988, Science 241:1077-1080), and RNase digestion of an RNA/DNA duplex (Winter qt al., 1985, Proc. Natl. Acad. Sci. 82:7575-7579) .

Now that applicants have shown that PZA resistance in *M. bovis* is conferred by an alteration the *pncA* gene and PZase, it is recognized for the first time that immunoassays can be used to distinguish *M. bovis* from *M. tuberculosis*. Therefore, the invention includes an isolated polyclonal or monoclonal antibody that preferentially binds the *M. bovis* PZase polypeptide. "Isolated antibodies" are those antibodies that are separated from the animal in which they were raised (e.g., a rabbit or mouse) or antibodies that were produced *in vitro.* Suitable immunoassays include Western blot analysis, slot or dot blot assays, ELISAs, immunoprecipitation assays, and the like.

"Antibody" means an immunoglobulin protein that is capable of binding an antigen. The term antibody is meant to include antibody fragments (e.g., F(Ab')₂, FAb', FAb) capable of binding the epitope or antigen of interest. The term "preferentially binds" means high avidity and/or high affinity binding of an antibody to a specific antigen or epitope. Antibody binding to an epitope on this specific antigen is stronger than binding of the same antibody to any other antigen or epitope. In particular, an antibody that preferentially binds *M. bovis* PZase binds *M. bovis* PZase more strongly than it binds *M. tuberculosis* PZase. In addition, an antibody that preferentially binds an antigen or epitope binds that antigen or epitope more strongly than it binds other molecules that may be present in the same sample as the antigen of interest. Antibodies that bind preferentially to a polypeptide of interest may be capable of binding other polypeptides at a weak, yet detectable, level (e.g., 10% or less of the binding shown to the antigen of interest). Such weak binding, or background binding, is readily discernible from the specific antibody binding to the polypeptide of interest, e.g., by use of appropriate controls.

Also included in the invention is a substantially pure *M. bovis* PZase polypeptide, or a conservative variant thereof. Such a polypeptide can be used to produce the above-described antibodies that are useful in immunoassays. The term "substantially pure polypeptide" means a preparation of a PZase polypeptide that is substantially free from the proteins and other naturally occurring organic molecules with which PZase is naturally associated. This typically means that the desired PZase polypeptide constitutes at least 60% of the dry weight of the preparation. Preferably, the preparation is at least 75%, more preferably at least 90%, and most preferably at least 99%, by weight, PZase polypeptide. A substantially pure PZase polypeptide may be obtained, for example, by extraction from a natural source (e.g., *M. bovis*) *;* by expression of a recombinant nucleic acid encoding a PZase polypeptide; or by chemical synthesis. Purity can be measured and/or obtained by any appropriate method, e.g., column chromatography, polyacrylamide gel electrophoresis, or HPLC.

A protein is substantially free of naturally associated components when it is separated from those contaminants that accompany it in its natural state. Thus, a protein that is chemically synthesized, or produced from a source different from the source from which the protein naturally originates, will be substantially free from its naturally associated components. Accordingly, substantially pure PZase includes recombinant PZase synthesized, for example, *in vitro* in a mammalian cell line, in *E. coli* or another single-celled microorganism, or in insect cells.

PCR-Sinqle-Strand Conformation Polvmorchism (PCR-SSCP)₋-. The preferred method for detecting an alteration in the *pncA* gene is "PCR-SSCP." In this method, a portion of the *pncA* gene is PCR amplified using primers that flank the alteration in the gene. The amplified DNA then is analyzed by gel electrophoresis under non-denaturing conditions. An alteration in the amplified DNA can be detected as an alteration in the mobility of the altered DNA through the gel, relative to the mobility of wild-type DNA. The term "amplifying" means to reproduce a nucleic acid, e.g., by DNA synthesis. For example, an amplified DNA molecule is one that reproduced such that the total number of copies of the particular DNA molecule is increased. Typically, amplification is accomplished by incubating a nucleic acid polymerase (e.g., DNA polymerase or Taq polymerase) with non-polymerized nucleotides (e.g., dATP, dCTP, dGTP, and dTTP), and a suitable buffer with a single-stranded nucleic acid template of the DNA to be amplified. For convenience, such amplification can be accomplished by "polymerase chain reaction" (PCR), in which a nucleic acid is synthesized in the presence of a thermostable polymerase (e.g., Taq polymerase), a large number of suitable nucleic acid primers and non-polymerized nucleotides (see Sambrook et al., *supra*). In a typical PCR reaction, (1) the strands of a double-stranded DNA molecule are separated by heating the DNA to produce single-stranded nucleic acid templates, (2) the temperature of the reaction is lowered, and the nucleic acid primers are annealed to single-strand nucleic acid templates, (3) DNA synthesis ensues, such that a double-stranded molecule is produced from each original single-stranded template, and steps (1-3) are repeated for numerous cycles (typically 35 cycles). Now that altered *pncA* genes have been identified and appreciated as indicators of PZA resistance, those of ordinary skill in the art of molecular biology can readily use DNA amplification techniques to amplify the *pncA* gene without undue experimentation.

Typically, the primers for PCR-SSCP are designed such that they can amplify a portion of the gene that is approximately 200 bp in length (e.g., a 100-500 bp, preferably, a 150-200 bp portion). Preferably, the altered nucleotide is approximately centered within the amplified sequence. The exact portion of the *pncA* gene that is amplified is not critical to the success of this method, provided that the amplified portion encompasses the alteration in the genomic nucleic acid that confers PZA resistance (e.g., in the case of *M. bovis,* the amplified portion should include the coding sequence of amino acid 57 of the PZase polypeptide). An example of a pair of primers that can be used to amplify a suitable portion of the *pncA* gene for identifying *M. bovis* is: and Sequences that are "substantially complementary" to these sequences also are useful for amplifying an appropriate portion of a nucleic acid of the mycobacterium that is being identified. This means that the primers must be sufficiently complementary to hybridize with their respective strands under conditions that allow the agent for polymerization to perform. In other words, the primers should have sufficient complimentarity with the 5' and 3' sequences flanking a portion of the mycobacterial nucleic acid that encompasses an the alteration that confers PZA resistance. The mycobacterial sequences to which the primers hybridize are considered the "target" flanking 5' and 3' polynucleotide sequences, as they flank the sequence that is to be amplified. Primers having "substantially the sequence" of the target polynucleotides differ in sequence from the target polynucleotide sequence, yet permit hybridization of the primer.

PCR amplification of the altered *pncA* gene can be carried out in accordance with conventional PCR-SSCP protocols (see Orita et al., *supra*)*.* For convenience, a labeled nucleotide (e.g., Sambrook, et al., Cold Spring Harbor Laboratory Press, 2nd Edition, (1989)) can be incorporated into the DNA that is amplified by PCR. Incorporation of a labeled nucleotide facilitates detection of the amplified DNA directly in a gel, and obviates the need for transferring the DNA from a gel onto a membrane. Once the DNA is amplified, it is denatured. Typically, denaturation will be accomplished by heating the DNA to 80-100°C for 5-10 minutes in the presence of formamide dye (95% formamide, 20 mM EDTA, 0.05% bromphenol blue, and 0.05% xylene cyanol). The heated, amplified DNA then is immediately cooled by incubating the DNA on ice for 5-10 minutes in order to prevent the DNA from renaturing.

In PCR-SSCP, the denatured, amplified DNA is electrophoresed on a gel under non-denaturing conditions. For amplified DNA fragments of approximately 200 bp, an example of a suitable gel is a 20% polyacrylamide/5% glycerol gel (16 x 20 cm) pre-chilled to 4°C and electrophoresed at 4°C at 5 W in 0.5 X TBE buffer. The electrophoresed DNA then can be detected by conventional methods (e.g., ethidium bromide staining, DNA hybridization, or in-gel detection of labeled DNA). Typically, the gel will include, as a control, DNA that is amplified from a known stock of *M. tuberculosis* and/or *M. bovis.* A detailed working example of this method is provided below.

The invention is suitable for distinguishing between *M.* bovis and *M. tuberculosis* in any of a variety of samples containing or believed to contain mycobacteria. For example, the mycobacteria can be contained within a biological fluid or tissue (e.g., sputum, blood, lung tissue) of a mammal (e.g., a human or cow). Alternatively, an *in vitro* culture of mycobacteria, (e.g., a tissue culture of mammalian cells that are infected with mycobacteria) can serve as the sample. Because PCR can be used to amplify a portion of the altered *pncA* gene, the sample can, in principle, contain a single mycobacterium. If desired, mycobacteria obtained from a mammal can be cultured *in vitro* according to conventional methods prior to assaying the mycobacteria for an altered *pncA* gene. Likewise, the *pncA* gene from a mycobacteria sample of interest can be cloned into a genetic vector prior to assaying for an alteration in the gene, if desired. In addition, any of the conventional methods for identifying *M. bovis* can be used in conjunction with this method for distinguishing *M. bovis* from *M. tuberculosis.*

### Use of Hybridization Methods to Identify M. bovis:

As an alternative to using PCR-SSCP to distinguish *M. bovis* from *M. tuberculosis*, traditional nucleic acid hybridization methods can be used to identify *M. bovis.* Thus, a nucleic acid probe(s) for use in a nucleic acid hybridization method(s) is included within the invention. Such a "probe" is a nucleic acid molecule (DNA or RNA) that includes a nucleotide sequence that is complementary to a portion of (i.e., capable of forming Watson-Crick base-pairs with part of) a wild-type or altered *pncA* gene, where the portion includes a nucleotide sequence encoding an aspartic acid residue at amino acid position 57 of the PZase polypeptide. A nucleic acid portion that is substantially complementary to the aforementioned probe is also useful as a probe, and thus is included within the invention. In a preferred embodiment, the invention includes a probe that is complementary to a portion of the *M. bovis pncA* gene that includes a G, rather than a C, at nucleotide 169. In another preferred embodiment, the probe is complementary to a portion of the *M. tuberculosis pncA* gene that includes a C at nucleotide 169. In other words, a probe that is complementary to either the wild-type or altered *pncA* genes is useful, and those skilled in the art will appreciate the results obtained with either type of probe.

Under typically stringent hybridization conditions, a probe that is complementary to the wild-type *pncA* gene will hybridize to (i.e., Watson-Crick base-pair with) the *pncA* gene of *M. tuberculosis,* but not the *pncA* gene of *M. bovis*. Thus, the inability of such a probe to hybridize to DNA in a sample indicates that the sample includes *M. bovis.* Inversely, a probe that includes a sequence that is complementary to the alteration in the *M. bovis pncA* gene will hybridize preferentially to the altered *M. bovis* gene rather than the wild-type *M. tuberculosis* gene. Those skilled in the art will know how to design appropriate controls for such assays (e.g., include a nucleic acid sample from a known mycobacterium and include a probe that hybridizes to a region of the *pncA* that is common to *M. tuberculosis* and *M. bovis*)*.* A typical probe consists of a chain of 8 to 20 ribonucleotides or deoxyribonucleotides, and is complementary to a portion of the *pncA* gene that is a chain of 8 to 20 deoxyribonucleotides. The position of the alteration along the length of the probe is not critical. If desired, the probes can be detectably labelled. Those of ordinary skill in the art will know or can readily ascertain various techniques for labelling nucleic acid probes.

Identification of PZA-resistant Mycobacteria: The invention also provides methods for identifying PZA-resistant mycobacteria generally. As discussed above, PZA-resistant strains of mycobacteria complicate efforts to treat and contain the spread of tuberculosis. Now that the molecular basis for PZA resistance has been revealed by the experiments described below, molecular-based assays can be used to identify PZA-resistant mycobacteria. Included are those mycobacteria that are naturally resistant to PZA, as well as those mycobacteria that acquire PZA resistance.

In principle, this aspect of the invention is a variation of the above methods for distinguishing between *M. bovis* and *M. tuberculosis.* For the first time, the scientific basis for PZA-resistant strains of mycobacteria has been discovered by applicants who have shown that these strains fail to encode a functional PZase polypeptide due to an alteration(s) in the *pncA* gene. Thus, as above for *M. bovis,* this aspect of the invention involves detecting an "altered" *pncA* gene in a mycobacterium as an indicator of PZA resistance.

As is described in detail in the examples below, several *pncA* gene alterations that confer PZA resistance have now been identified. These alterations are summarized in Table 1.

**TABLE 1 Mutations in the pncA Gene of PZA-resistant M. tuberculosis**

| Strain | *pncA* Mutation | PZA susceptibility^{a} | MIC For PZA^{b} | MDR^{c} |
|---|---|---|---|---|
| PZA-R | Deletion of G 288^{d} | - | >500 µg/ml | - |
| Vertullo | Deletion of G 162 | - | >500 µg/ml | + |
| F36946 | Asp 63 - His | - | >500 µg/ml | + |
| M3S169 | Gln 141 → Pro | - | >500 µg/ml | + |
| M36470 | Cys 138 - Ser | - | >500 µg/ml | + |

| | | | | |
|---|---|---|---|---|
| ^{a} Determined according to the method of McDermott and Tomsett, 1954, Am. Rev. Tuberc. 70:748-754 | | | | |
| ^{b} MIC: Minimal Inhibitory Concentration | | | | |
| ^{c} MDR: Multi-Drug Resistance against INH and rifampicin | | | | |
| ^{d} The amino acid positions referred to herein are numbered with respect to the full-length wild-type *M. tuberculosis* PZase polypeptide. The position of these amino acids may be different in altered PZase polypeptides. | | | | |

In each of strains PZA-R and Vertullo, the deletion of a nucleotide results in a "-1 frameshift" in the translational reading frame of the mRNA encoded to the genes. In other words, because of the -1 frameshift at position 288, the *pncA* gene of the PZA-R strain encodes proline-valine-threonine (and so forth), rather than proline-glycine-aspartic acid at residues 54-56. Similarly, because of the -1 frameshift at position 162, the *pncA* gene of the Vertullo strain encodes lysine-cysteine-leucine (and so forth), rather than lysine-glycine-alanine at residues 96-98.

Now that alterations in the *pncA* gene of PZA resistant mycobacteria have been identified and shown to be responsible for PZA resistance, PZA-resistant strains of mycobacteria can readily be identified by employing methods analogous to those described above for identifying *M. bovis.* In addition, new PZA-resistant strains having as yet unidentified specific alterations the *pncA* gene can be readily identified by those of ordinary skill without resort to undue experimentation. For example, where a mycobacterium is found to be PZA resistant, but does not contain one of the particular alterations in the PZA gene described herein, it is reasonable that such mycobacterium contains a new alteration in its PZA gene. This alteration can be readily and specifically identified, for example, by nucleic sequencing and comparing the sequence determined to the wild-type *pncA* nucleotide sequence.

As is the case for identifying *M. bovis,* PCR-SSCP is a preferred method for identifying PZA-resistant mycobacteria because this method is rapid and reproducible. Included within the invention is a series of overlapping primers that is particularly useful for detecting alterations in the *pncA* gene by PCR-SSCP. These primers permit amplification of the entire *pncA* gene; thus, they are useful for identifying any alteration in the *pncA* gene. These primers include and and sequences substantially complementary thereto. The relationship between these primers and the *pnc*A gene is shown schematically in Fig. 2; the portion of the *pnc*A gene that is amplified by particular pairs of primers is indicated.

Now that alterations in the *pncA* gene have been identified and recognized as conferring PZA resistance to mycobacteria, nucleic acid hybridization methods can also be used to identify PZA-resistant mycobacteria. Accordingly, the invention includes a nucleic acid probe(s) (DNA or RNA) for identifying a PZA-resistant mycobacterium. The probe (typically 8-20 nucleotides) includes a nucleic acid that is complementary to a portion (typically 8-20 nucleotides) of an altered *pncA* gene of a PZA-resistant mycobacterium, where the portion confers PZA-resistance. Included are nucleic acid probes that are complementary to a portion of the *pncA* gene that (i) results in a -1 frameshift at amino acid position 96; (ii) results in a -1 frameshift at amino acid position 126; (iii) encodes a histidine residue at amino acid position 63; (iv) encodes a serine residue at amino acid position 138; and (v) encodes a proline residue at amino acid position 141. Specific examples include those probes that are complementary to a portion of an altered *pncA* gene that comprises (i) a deletion of nucleotide 288; (ii) a deletion of nucleotide 162; (iii) a change of G to C at nucleotide 187; (iv) a change of T to A at nucleotide 412; and (v) a change of A to C at nucleotide 422. These probes, when used in hybridization methods, will hybridize preferentially to altered *pncA* genes. Thus, these probes can be used to distinguish PZA-resistant mycobacteria from PZA-sensitive mycobacteria by hybridizing the probes to nucleic acids (i.e., DNA or RNA) of the mycobacteria under stringent hybridization conditions. Probes that are complementary to the wild-type *pncA* sequences can also be used to detect PZA-resistant mutants when the probes are designed to include a sequence that confers PZA resistance when the sequence is altered.

The deletion of a single nucleotide in each of the PZA-R and Vertullo strains of *M. tuberculosis*, described above, results in -1 frameshift during synthesis of the altered PZase polypeptide encoded by the altered *pncA* gene. Based upon the nucleotide sequence of the *pncA* gene of the PZA-R strain, the -1 frameshift results in a PZase polypeptide that is 126 amino acids in length, rather than a full-length 186 amino acids. Similarly, the -1 frameshift of Vertullo results in a truncated PZase polypeptide of 117 amino acids. These PZA-resistant strains of mycobacteria and their homologs can also be detected in immunoassays that employ antibodies, especially monoclonal antibodies, that specifically bind the carboxy-terminal portion of wild-type PZase that is missing in the truncated PZase polypeptides. Thus, the altered PZase polypeptides of PZA-resistant mycobacteria having the alterations fail to be bound by such antibodies, even though the altered PZase polypeptides would be bound by antibodies that specifically bind epitopes at the amino-terminal portion of the polypeptides.

In principle, immunoassays can also be easily developed and used to identify PZA-resistant strains of mycobacteria, because a single amino acid alteration also can produce an epitope(s) that is specific to the altered PZase polypeptide. Also, a truncated, or even elongated, PZase polypeptide that arises from a frameshift can have an amino acid sequence that, at least in part, differs significantly from that of wild-type *M. tuberculosis* PZase polypeptide. Such is the case for the PZase polypeptides of the Vertullo and PZA-R strains of *M. tuberculosis* because of the -1 frameshifts resulting from the deletion of nucleotides 162 and 288, respectively. Thus, these PZase polypeptides can be distinguished from wild-type PZA in immunoassays that employ antibodies that preferentially bind the portion of the polypeptide that is altered as a result of the translational frameshift.

Accordingly, the invention includes isolated antibodies useful in performing immunoassays for identifying a PZA-resistant mycobacterium. In particular, the invention includes an isolated polyclonal or monoclonal antibody that preferentially binds an altered PZase polypeptide that confers PZA resistance, such as a PZase polypeptide of an *M. tuberculosis* strain homologous to an *M. tuberculosis* strain selected from the group consisting of M36470, M3S169, F36946, Vertullo, and PZA-R. The term "homologous" is meant to include strains of mycobacteria that have PZase polypeptides that are identical to, or conservative variations of, the PZase polypeptides of the recited strains. Such homologous strains of mycobacteria can differ from the recited strains at any genetic locus, provided that the homologous strain is capable of causing respiratory tuberculosis in mammals, and is rendered PZA-resistant by an altered *pncA* gene. Included by the term "homologous" are the recited strains themselves, namely, M36470, M3S169, F36946, Vertullo, and PZA-R.

The invention also includes a substantially pure altered PZase polypeptide that confers PZA resistance, or a conservative variant thereof. Specific examples of such polypeptides are the PZase polypeptides of the M36470, M3S169, F36946, Vertullo, and PZA-R strains of *M. tuberculosis* and strains homologous thereto. In other words, any PZase polypeptides having the alterations summarized in Table 1 are expected to confer PZA resistance, and are included within the invention.

In another aspect, the invention provides a method for treating a mammal that is infected with PZA-resistant mycobacteria (e.g., *M. bovis* or PZA-resistant *M. tuberculosis*)*;* thus, this aspect of the invention provides a method for treating tuberculosis. The method involves introducing a *pncA* gene that encodes a functional PZase polypeptide into at least a portion (preferably at least 10%, more preferably at least 50%, and most preferably at least 75%) of the PZA-resistant mycobacteria that infect the mammal. A PZase polypeptide is considered "functional" if it retains a biological activity of the full length PZase polypeptide, namely the ability to convert PZA to pyrazinoic acid. Expression of the *pncA* gene in the PZA-resistant mycobacteria renders the mycobacteria sensitive to PZA. The mammal then is administered a therapeutically effective amount of PZA, which inhibits or kills at least a portion of the mycobacteria that infect the mammal. A treatment that "inhibits" the mycobacteria slows or prevents the growth or reproduction of the bacteria inside the infected mammal and/or decreases or prevents the production of release of mycobacterial toxins into the cells of the infected mammal. Generally, the terms "treating," "treatment," and the like are used herein to mean obtaining a desired pharmacologic and/or physiologic effect. The effect may be prophylactic in terms of completely or partially preventing a mycobacterial infection or disease (e.g., tuberculosis) or sign or symptom thereof, and/or may be therapeutic in terms of a partial or complete cure for an infection or disease and/or adverse effect attributable to the infection or disease. "Treating" as used herein covers any treatment of an infection or disease in a mammal, particularly a human, and includes:
(a) preventing the disease from occurring in a subject that may be predisposed to the disease, but has not yet been diagnosed as having it;
(b) inhibiting the infection or disease, i.e., arresting its development; or
(c) relieving or ameliorating the infection or disease, i.e., cause regression of the infection or disease. The invention is thus directed to treating patients who are afflicted with a mycobacterial infection or tuberculosis. More specifically, "treatment" is intended to mean providing a therapeutically detectable and beneficial effect on a patient suffering from a mycobacterial infection or tuberculosis.

The term "introducing" a *pncA* gene into a mycobacterium means to insert a *pncA* gene into the mycobacterium such that the *pnc*A gene is available to be expressed by the cellular machinery (e.g., polymerases and ribosomes) of the mycobacterium. The *pncA* gene can be introduced into mycobacteria by using standard recombinant DNA techniques to insert the *pncA* gene into a mycobacteriophage, such as mycobacteriophage L5, L1, or D29 (Hatfull and Jacobs, Mycobacteriophages: Cornerstones of Mycobacterial Research. In Tuberculosis. (ed) B.R. Bloom, ASM Press, Washington, DC). A therapeutically effective amount of the *pncA* gene then is administered to the infected mammal, and a therapeutically effective amount of PZA is administered to the mammal. The term "therapeutically effective" means sufficient *pncA* gene to produce PZase to thereby render the mycobacteria resistant to PZA. It is believed that one copy of the *pncA* gene per organism would render the organism sensitive to PZA. Various means can be used to "administer" PZA or a *pncA* gene to a mammal as a therapeutic. Generally, the therapeutic to be delivered can be formulated into a pharmaceutical composition by admixture with a pharmaceutically acceptable non-toxic excipient or carrier (e.g., saline). In practicing the invention, the therapeutic can be prepared for use in parenteral administration (e.g., for intravenous injection or intra-arterial injection). Preferably, the *pncA* gene is administered to the mammal intranasally or intrabronchially, e.g., as a liquid or aerosol formulation (e.g., nasal drops or spray) in a pharmaceutically acceptable excipient. Preferably, PZA is administered orally or according to other conventional methods. While the optimal therapy regime can be determined for each treated mammal (e.g., a human or cow), a typical regime involves administration of 10⁶ to 10⁸ pfc of mycobacteriophage carrying the *pncA* gene, 1 to 7 times weekly for a period of 3 to 12 (preferably 6-12) months, or until the signs and symptoms of the mycobacterial infection or tuberculosis are ameliorated. PZA typically is administered daily (in combination with isoniazid and rifampicin, if desired) for the first two months although alternative PZA treatment regimes can be used (Mitchison et al., 1985, Tubercle 66: 219-225). If desired, this therapy regime can be used in conjunction with conventional methods, such as administration of at least one other mycobacterial therapeutic agent (e.g., isoniazid and/or rifampicin).

Included within the invention is a kit(s) for distinguishing *M. bovis* from *M. tuberculosis* or for identifying PZA-resistant mycobacteria. Such a kit is a package that carries a container(s) that contains any or all of the primers and probes described above. Preferably, the kit also contains isolated nucleic acid(s) corresponding to all or a portion of the wild-type *M. tuberculosis pncA* gene and/or an altered *pncA* gene (e.g., from *M. bovis* or one of the acquired PZA-resistant *M. tuberculosis* strains, such as Vertullo). A kit can contain the above-described monoclonal or polyclonal antibodies useful for identifying PZA-resistant mycobacteria. Typically, the nucleic acids, antibodies, and polypeptides that are contained within the kits are present in a measured amount that is indicated on a label(s) on the container within the kit. In addition, a typical kit includes a set of instructions for practicing the methods of the invention. Nucleic acids, antibodies, and polypeptides that are useful as controls can also be included in a kit of the invention.

Summary of the Examples**:** Several working examples are provided below. These examples describe (i) the cloning of the *M. tuberculosis pncA* gene, (ii) identification of the alterations in the *pncA* gene that confer PZA resistance, (iii) use of the *pncA* gene to render PZA-resistant *M. tuberculosis* sensitive to PZA, (iv) use of PCR-SSCP to rapidly differentiate between *M. bovis* and *M. tuberculosis*, and (v) use of PCR-SSCP to identify PZA-resistant *M. tuberculosis* strains. Before describing the examples in further detail, a description of the materials and methods used in these examples is provided.

### Materials and Methods

Mycobacterial strains and DNA. Strains of *M. tuberculosis, M. bovis,* and BCG were grown in 7H9 liquid medium with ADC (Albumin-Dextrose-Catalase) enrichment at 37°C for 2-4 weeks. The PZA-resistant *M. tuberculosis* strain (ATCC 35828) derived from type strain H37Rv was obtained from American Type Culture Collection. PZA-resistant clinical isolates were provided by L. Heifets (National Jewish Center for Immunology and Respiratory Diseases, Denver, Colorado), J. Belisle and P. Brennan (Colorado State University). Genomic DNA from *M. tuberculosis* strains and other mycobacterial species was isolated as described previously (Zhang, et al., Infect. Immun. 60, 2160-2165 (1992)). Genomic DNA from *M. bovis* strains was provided by V.P. Shankar (Texas A & M University Health Science Center).

Cloning and characterization of the *M. tuberculosis pncA* gene. To clone the *M. tuberculosis pncA* gene, degenerate primers were designed based on the putative *E. coli* nicotinamidase amino acid sequence (Jerlstrom, et al., Gene 78, 37-46 (1989)). When cloned into a plasmid vector, the putative *E. coli pncA* indeed expresses functional nicotinamidase activity upon transformation into a *Salmonella typhimurium pncA* mutant strain JF49 (Foster et al., J. Bacteriol., 137, 1165-1175 (1979)). The forward primer was designed from the 9-17 amino-acid residues VDLQNDFCA (SEQ ID NO: 13), and the reverse primer from the 168-179 amino-acid residues GYKVNVITDGC (SEQ ID NO: 14), of the *E. coli* enzyme (Jerlstrom, et al., Gene 78, 37-46 (1989)). PCR was performed using the above primers with genomic DNA from a PZA-susceptible *M. tuberculosis* strain as template (Saiki, et al., Science 239, 487-491 (1988)). A 500 bp PCR product was obtained, and sequence analysis showed that it contained a partial open reading frame with homology to the *E. coli* nicotinamidase gene described above.

A cosmid clone containing the complete *M. tuberculosis pncA* gene was isolated using the ³²P-labelled 500 bp PCR product containing the partial *pncA* gene as a probe, by screening an integrating mycobacterial shuttle cosmid DNA library constructed from the *M. tuberculosis* Erdman strain (provided by W. R. Jacobs, Albert Einstein College of Medicine, New York). Restriction mapping of the cosmid DNA, followed by Southern blot analysis using the 500 bp PCR product as a probe, and transformation studies localized the *M*. *tuberculosis pncA* gene on a 3.2 kb *Eco*RI-*Pst*I fragment, which subsequently was subcloned into pUC19. The standard molecular biology techniques were carried out as described previously (Sambrook, et al., Cold Spring Harbor Laboratory Press, Cold Spring Harbor, New York, 1989).

The complete *M. tuberculosis pncA* sequence (from *M. tuberculosis* Erdman strain) (accession number under application) was determined from the 3.2 kb *Eco*RI*-Pst*I fragment using primers derived from sequences of the 500 bp *pncA*-containing PCR product. The determination of *pncA* sequences from *M. bovis*, BCG and other *M. tuberculosis* strains was performed by PCR direct sequencing using primers designed from the above-described complete *M. tuberculosis pncA* sequence in an automatic DNA sequencer. The sequence homology alignment between *M. tuberculosis* and *E. coli* PncA sequences was performed using a FASTA algorithm.

Transformation of tubercle bacilli. The *pncA* plasmid construct for transformation of PZA-resistant BCG and H37Rv was made as follows. The 3.2 kb *Eco*RI-*Pst*I fragment containing the functional *M. tuberculosis pncA* was cloned into the hygromycin mycobacterial shuttle vector p16R1 (Garbe, et al., Microbiology 140, 133-138 (1994)) as described (Sambrook, et al., Cold Spring Harbor Laboratory Press, Cold Spring Harbor, New York, 1989). Both the p16R1 3.2 kb *pncA* construct and the cosmid DNA containing the functional *M. tuberculosis pncA* gene, along with the vector controls, were transformed by electroporation into the naturally PZA-resistant *M. bovis* BCG and PZA-resistant *M. tuberculosis* H37Rv as described (Zhang, et al., Mol. Microbiol. 88, 521-524 (1993)).

PZase enzyme assay. Pyrazinamidase activity was assayed according to the method of Wayne (Wayne, Am. Rev. Respir. Dis. 109, 147-151 (1974)). Briefly, a heavy bacterial inoculum (several loopfuls) was inoculated onto the surface of Dubos agar (Difco) containing 0.1 mg/ml PZA in a test tube, which was then incubated at 37°C for 4 days. Two milliliters of 1% ferrous ammonium sulfate were added, and the test tubes were incubated at 4°C for 1-2 hours. A positive PZase activity appeared as a brownish band in the agar surface. A positive culture (PZA-sensitive *M. tuberculosis* strain H37Rv) and a negative culture (BCG Pasteur) were included as controls.

PZA susceptibility testing. The susceptibility to PZA of recombinant mycobacterial strains was tested in 7H9 liquid medium adjusted to acid pH (5.6) as described elsewhere (McDermott, et al., Am. Rev. Tuberc. 70, 748-754 (1954)). Briefly, 2 week old liquid mycobacterial cultures (about 10⁷⁻⁸ bacilli) were diluted at 10⁻³ and 10⁻⁵ in acid 7H9 liquid medium (pH 5.6) containing 50, 100, 200, 500, 1000 µg/ml PZA in Eppendorf tubes. Each strain tested for PZA susceptibility was in duplicate. The Eppendorf tubes were then incubated at 37°C for 2-3 weeks when the extent of growth inhibition was assessed. PZA susceptible H37Ra or H37Rv, and PZA-resistant BCG were included as sensitive and resistant controls, respectively.

Macrophage infections with mycobacteria and assay of PZA susceptibility of intracellular mycobacteria were performed as described (Skinner, et al., Antimicrob Agent Chemother, 38, 2557-2563 (1994)). Briefly, 5 X 10⁵ J774 murine macrophages were infected with 2-5 X 10⁶ bacteria in 24-well tissue culture plates. Each mycobacterial strain used for macrophage infection was in triplicate. After infection at 37°C for 1-2 hours, the extracellular bacteria were washed off with PBS twice. Tissue culture medium (RPMI 1640) containing various concentrations of PZA or streptomycin was added to the infected macrophages and the plates were incubated in a CO₂ incubator at 37°C for 1-2 weeks. The number of intracellular bacteria was counted under the microscope and expressed as an average of at least 50 infected macrophages per well for all three triplicate wells for each bacterial strain.

### EXAMPLES

Cloning and characterization of the *M. tuberculosis pncA* gene. The *M. tuberculosis pncA* gene was cloned by PCR using degenerate primers based on the amino acid sequence derived from the *E. coli* nicotinamidase gene (*pncA*) (Jerlstrom, et al., Gene 78, 37-46 (1989)). The *M. tuberculosis pncA* was initially cloned on a 500 bp PCR product from a PZA-susceptible *M*. *tuberculosis* strain. Sequence analysis showed that the PCR product contained a partial open reading frame with homology to the *E. coli* nicotinamidase sequence (Jerlstrom, et al., Gene 78, 37-46 (1989)). To obtain the complete *M. tuberculosis pncA* gene, an *M. tuberculosis* integrating cosmid DNA library was screened using the 500 bp *pncA*-containing PCR product as a probe. A pncA-hybridizing cosmid clone was isolated and used to transform BCG, an attenuated vaccine strain derived from *M. bovis,* in order to confirm the identity of the putative *M. tuberculosis pncA* gene. Indeed, the cosmid DNA containing the putative *M. tuberculosis pncA* gene conferred PZase activity to BCG, a natural mutant defective in PZase. The integration of the *pncA* cosmid DNA into *M. bovis* BCG genome was confirmed by Southern analysis. The functional *M. tuberculosis pncA* gene was localized on a 3.2 kb *Eco*RI-*Pst*I fragment (Fig. 3A), by restriction mapping of the cosmid DNA in combination with BCG transformation studies using DNA constructs derived from the cosmid DNA insert. To confirm that the PZase activity is due to the *pncA* gene, but not to other DNA in the *pnc*A upstream region on the 3.2 kb *Eco*RI-*PstI* fragment, BCG was transformed with the 2.3 kb *Eco*RI-*Sma*I DNA construct that contains the *pncA* upstream region and partial *pnc*A gene; however, no PZase activity was detectable in the BCG transformant. Sequence analysis of the 2.3 kb *Eco*RI*-Sma*I fragment does not show significant open reading frames with homology to other known proteins in the database. Transformation of BCG with a construct that contains the *pncA* gene alone with its 120 bp upstream sequence as a PCR fragment gave functional expression of PZase activity, indicating that the PZase activity was indeed conferred by the *pncA* gene.

Sequence analysis revealed that the *M. tuberculosis pncA* gene (558 bp) encoded a protein of 186 amino acids (Fig. 1), with 35.5% overall amino acid identity to the E. coli nicotinamidase (Jerlstrom, et al., Gene 78, 37-46 (1989)) (Fig. 3B). The predicted molecular mass of the *M. tuberculosis* PZase is approximately 20 kilodaltons (kD), and is smaller than the E. coli homolog which consists of 213 amino acids with a size of 23 kD (Jerlstrom, et al., Gene 78, 37-46 (1989)).

Identification of mutations in the *pncA* gene of PZA-resistant strains. Using the 3.2 kb *Eco*Rl-*Pst*I *pncA* containing DNA fragment as a probe, Southern blotting analysis was performed on a panel of 8 PZA-resistant *M. tuberculosis* strains, 3 *M. bovis* strains and 3 BCG substrains. None of these strains had any gross deletions of the *pncA* gene or restriction fragment length polymorphism. *M*. *bovis*, as a species, lacks PZase (Konno, et al., Nature, 184, 1743-1744 (1959)) (Konno, et al., Am Rev. Respir. Dis. 95, 461-469 (1967), yet it nonetheless had the *pncA* gene, as evidenced by the same sized hybridization fragment as that in *M. tuberculosis.* The *M. tuberculosis pncA* gene did not hybridize with genomic DNA from mycobacterial species that do not cause respiratory tuberculosis, such as *M.* smegmatis, *M. vaccae* and *M. kansasii.*

The sequence of the *pncA* gene from naturally PZA-resistant *M. bovis* and an a panel of acquired PZA-resistant *M. tuberculosis* strains was determined. Surprisingly, three *M. bovis* strains (Ravenel, and two veterinary isolates) and three BCG substrains (Pasteur, Copenhagen, Glaxo) all had the same single point mutation in the *pncA* gene, changing from "C" to "G" at nucleotide position 169, which caused a substitution of Histidine (CAC) for Aspartic Acid (GAC) at amino acid position 57 of the PZase polypeptide (Fig. 1). Because no other mutations were found in the *pncA* gene, this particular substitution is concluded to have caused the defective PZase in these *M. bovis* and BCG strains. Consistent with this conclusion is the observation that, the BCG strains transformed with the 3.2 kb *Eco*RI*-Pst*I construct containing the functional *M. tuberculosis pncA* gene restored PZase sensitivity.

Of the 8 sequenced *M. tuberculosis* strains that are PZA-resistant in conventional tests, 5 strains had point mutations within the *pncA* gene (see Table 1 above). The H37Rv, CSU20, and CSU25 strains lacked mutations in the *pncA* gene. The PZA-resistant strain PZA-R (ATCC 35828, PZase-negative) derived from H37Rv, and one MDR strain "Vertullo" each had a single nucleotide "G" missing at nucleotide positions 288 and 162, respectively, leading to premature termination that caused truncated polypeptides with no PZase activity. Three other PZA-resistant clinical isolates of *M. tuberculosis* (MDR strains) contained missense mutations in the *pncA* gene, causing substitutions at the following amino acid positions: Asp63 (GAC) --> His (CAC), Cys138 (TGT) --> Ser (AGT), Gln141 (CAG) --> Pro (CCG). These data suggest that these substitutions are responsible for PZA-resistance and the defective PZase enzyme activity in these PZA-resistant strains. Of the 3 apparently PZA-resistant strains (CSU20, CSU25, and H7728) that did not have mutations in the *pncA* gene, re-testing of the minimal inhibitory concentration (MIC) of PZA indicated that these three strains are "false resistant," and are in fact susceptible to PZA with positive PZase activity.

Use of PCR-SSCP to distinguish *M. bovis* from *M*. *tuberculosis*. In this example, PCR was performed using the following cycling parameters: 95°C for 5 minutes, followed by 30 cycles of (95°C for 1 minute, 55°C for 1 minute, and 72°C for 1 minute). The PCR reaction included 1 X PCR Buffer (GIBCO BRL), dNTPs at 200 *µ* mole, primers at 0.1 *µ*g, mycobacterial genomic DNA 0.1-0.5 *µ*g (V.P. Shankar; Texas A & M University Health Science Center), and 2.5 units of Tag DNA polymerase (GIBCO BRL). The primers used for detection of *M. bovis* were: and corresponding to nucleotides 91-110 and 270-251, respectively, of the *pnc*A gene. When used together, these primers amplify a 180 bp DNA fragment.

Polyacrylamide gel electrophoresis was used to show the single strand conformation polymorphism in the amplified DNA. The PCR products (10 *µ*l, containing 0.5-1.0 µg DNA) were denatured by boiling for 5-10 minutes in formamide dye. The boiled DNA was immediately incubated on ice for 5-10 minutes. The denatured DNA then was electrophoresed on a 20% polyacrylamide/5% glycerol gel (16 x 20 cm) that was pre-cooled to 4°C. The gel was electrophoresed in 0.5 X TBE buffer at a constant power of 5 W at 4°C overnight. The gel then was stained with ethidium bromide (0.5 µg/ml), and the SSCP bands were visualized under UV light. As is illustrated in Fig. 4, the mobility of DNA corresponding to *M. bovis* differs from the mobility of DNA corresponding to *M. tuberculosis*. Thus, *M. bovis* strains can be rapidly differentiated from *M. tuberculosis* by PCR-SSCP, based on the detection of a characteristic *M. bovis* alteration in the *pncA* gene.

Use of PCR-SSCP to Identify PZA-resistant Strains of *M. tuberculosis.* In this example, PCR-SSCP was performed essentially as described above, except that the primers and mycobacterial DNA differed. The primers used were: and By using these primers in pairs (P3 with P4; P5 with P6; and P7 with P8), overlapping portions (≈200 bp) of the *pncA* gene are amplified, which together correspond to nearly the entire *pncA* gene (Fig. 2). By using a set of primers that amplify several portions of the *pncA,* one can use PCR-SSCP to identify a PZA-resistant mycobacterium, even if one does not know precisely where in the *pncA* gene an alteration is located.

As was the case for *M. bovis* above, polyacrylamide gel electrophoresis was used to show the single strand conformation polymorphism in the amplified DNA after the DNA was denatured and cooled on ice. As is illustrated in Fig. 5, the mobility of DNA corresponding to PZA-resistant *M. tuberculosis* differs from the mobility of DNA corresponding to wild-type (PZA-sensitive) *M. tuberculosis*. Thus, PZA-resistant strains of *M. tuberculosis* can be rapidly identified by PCR-SSCP.

Transformation of PZA-resistant strains with a functional *pnc*A gene. As is shown below, introduction of a functional *pncA* gene into a PZA-resistant *M. bovis* or *M. tuberculosis* renders the mycobacterium PZA-sensitive. In this case, the susceptibility of *M. bovis* BCG and the *M. tuberculosis* strain PZA-R were tested. The *M. bovis* BCG is a naturally PZA-resistant strain, and the *M. tuberculosis* strain PZA-R is an acquired resistance strain derived from H37Rv. These two PZA-resistant strains were transformed with the 3.2 kb *Eco*RI-*Pst*I plasmid construct that contains the functional *pncA* gene. In both cases, the resulting *pncA* transformants expressed PZase enzyme activity and became susceptible to PZA (MIC = 50 *µg* ml⁻¹) at acid pH (5.5) *in vitro.* In contrast, BCG and PZA-R vector control strains remained PZA-resistant (MIC > 1000 *µg* ml⁻¹) and PZase-negative.

The *pncA* gene can also be used to render intracellular *M. tuberculosis* susceptible to PZA. In this case, the recombinant *M. bovis* was tested for PZA-susceptibility while inside macrophages. Indeed, the recombinant BCG expressing the *M. tuberculosis* PZase became susceptible to PZA in J774 macrophages. By contrast, the control BCG remained resistant to PZA at various drug concentrations (Table 2). At high PZA concentrations (500 *µ*g ml⁻¹), the control BCG was also inhibited slightly, but not as much as the recombinant BCG. In a parallel control experiment with streptomycin, both the recombinant BCG and the vector control BCG were equally inhibited by streptomycin in J774 macrophages (Table 2), indicating that the PZA susceptibility of the recombinant BCG is due to expression of the PZase activity conferred by the *M. tuberculosis pncA* gene.

**Table 2 PZA Susceptibility of Recombinant BCG in J774 Macrophage**

| Drugs | Drug concentration (*µ*g/ml) | Number of bacilli per macrophage | |
|---|---|---|---|
| | | BCG recombinant (PZase⁺) | BCG vector (PZase⁻) |
| PZA | 0 | >20 | >20 |
| | 25 | 7 | >20 |
| | 200 | 5 | 15 |
| | 500 | 2 | 15 |
| Streptomycin | 0 | >20 | >20 |
| | 25 | 8 | 9 |
| | 50 | 4 | 7 |
| | 100 | 4 | 4 |

## Claims

1. A method for differentiating between *M. tuberculosis* and *M. bovis* in a sample, the method comprising detecting in the sample an altered *pncA* gene, wherein the altered *pncA* gene encodes an altered PZase polypeptide comprising a change from a histidine residue to an aspartic acid residue at amino acid position 57 compared to the wild-type *M. tuberculosis* PZase having the sequence of SEQ m NO. 2, and the altered *pncA* gene indicates that the sample comprises *M. bovis.*

2. The method of claim 1, wherein the change from histidine to aspartic acid at amino acid position 57 results from the change of C to G at nucleotide 169 of the altered *pncA* gene compared to the wild-type *M. tuberculosis pncA* gene having the sequence of SEQ m NO. 1.

3. The method of claim 1 or 2, wherein the detecting comprises:
a) amplifying a region of the nucleic acid of the mycobacteria by means of a pair of oligonucleotide primers that hybridize to target flanking 5' and 3' polynucleotide sequences of the nucleic acid, wherein said target flanking 5' and 3' polynucleotide sequences are: and and sequences complementary thereto; and
b) detecting the amplified region.

4. The method of claim 3, wherein the amplifying is by polymerase chain reaction (PCR).

5. The method of claim 1, wherein the altered *pncA* gene is detected by PCR-single strand conformation polymorphism (PCR-SSCP), single-strand conformation polymorphism (SSCP), DNA sequencing, nucleic acid hybridization, denaturing gradient gel electrophoresis, ligase mediated gene detection or RNase digestion of an RNA/DNA duplex.

6. A method for identifying a pyrazinamide (PZA)- resistant mycobacterium, the method comprising detecting an altered *pncA* gene compared to the wild-type *M. tuberculosis pncA* gene in the mycobacterium, wherein the altered *pncA* gene confers PZA resistance and wherein the wild-type *M. tuberculosis pncA* gene encodes the wild-type *M. tuberculosis* PZase having the sequence of SEQ ID NO. 2.

7. The method of claim 6, wherein the altered *pncA* gene is amplified.

8. The method of claim 7, wherein the altered *pncA* gene is amplified by polymerase chain reaction (PCR).

9. The method of claim 8, wherein the altered *pncA* gene is deleted by PCR-single strand conformation polymorphism (PCR-SSCP), single-strand conformation polymorphism (SSCP), DNA sequencing, nucleic acid hybridization, denaturing gradient gel electrophoresis, ligase mediated gene detection or RNase digestion of an RNA/DNA duplex.

10. The method according to any of claims 6 to 9. wherein the mycobacterium is *M. tuberculosis**.***

11. The method according to any of claims 6 to 9. wherein the alteration in the altered *pncA* gene is chosen from the group consisting of.
- the alteration in the altered *pncA* gene encodes an aspartic acid residue at amino acid position 57 compared to the wild-type PZase polypeptide of *M. tuberculosis* having the sequence of SEQ ID NO. 2.
- the alteration in the altered *pncA* gene comprises a change from C to G at nucleotide 169 compared to the the wild-type *pncA* gene having the sequence of SEQ ID NO. 1,
- the alteration in the altered *pncA* gene is a -1 frameshift in the translational reading frame of the mRNA-corresponding to amino acid position 96 of wild -type PZase polypeptide of *M. tuberculosis* having the sequence of SEQ ID NO. 2,
- the alteration in the altered *pncA* gene comprises a deletion of nucleotide 288 of the wild-type *pncA* gene of *M. tuberculosis* having the sequence of SEQ ID NO.1.
- the alteration in the altered *pncA* gene is a -1 frameshift in the translational reading frame of the mRNA corresponding to amino acid position 54 of wild-type PZase polypeptide of *M. tuberculosis* having the sequence of SEQ ID NO. 2,
- the alteration in the altered *pncA* gene comprises a deletion of nucleotide 162 of the wild-type *pncA* gene of *M. tuberculosis* having the sequence of SEQ ID NO.1,
- the alteration in the altered *pncA* gene encodes a histidine residue at amino acid position 63 of wild-type PZase polypeptide of *M. tuberculosis* having the sequence of SEQ ID NO. 2,
- the alteration in the altered *pncA* gene comprises a change of G to C at nucleotide 187 of the wild-type *pncA* gene of *M. tuberculosis* having the sequence of SEQ ID NO. 1,
- the alteration in the altered *pnc*A gene encodes a serine residue at amino acid position 138 of wild-type PZase polypeptide of *M. tuberculosis* having the sequence of SEQ ID NO. 2,
- the alteration in the altered *pncA* gene comprises a change of T to A at nucleotide 412 of the wild-type *pncA* gene of *M*. *tuberculosis* having the sequence of SEQ ID NO. 1,
- the alteration in the altered *pncA* gene encodes a proline residue at amino acid position 141 of wild-type PZase polypeptide of *M. tuberculosis* having the sequence of SEQ ID NO. 2, and
- the alteration in the altered *pncA* gene comprises a change of A to C at nucleotide 422 of the wild-type *pncA* gene of *M. tuberculosis* having the sequence of SEQ ID NO. 1.

12. An isolated nucleic acid encoding a wild-type *M. tuberculosis* pyrazinamidase (PZase) polypeptide **characterized by** SEQ ID NO. 2, or conservative variants thereof having the biological activity of SEQ ID NO. 2, or a nucleic acid encoding an altered *M*. *tuberculosis* PZase polypeptide compared to wild-type *M. tuberculosis* PZase having SEQ ID NO. 2. and conferring PZA resistance.

13. The nucleic acid of claim 12, wherein the nucleic acid has the nucleotide sequence of Fig. 1 (SEQ ID NO: 1), or degenerate variants thereof, and encodes the amino acid sequence of Fig. 1 (SEQ ID NO: 2) corresponding to the wild-type *M. tuberculosis* PZase.

14. An isolated nucleic acid encoding *M*. *bovis* PZase polypeptide having an aspartic acid residue at amino acid position 57 relative to SEQ ID NO. 2.

15. The isolated nucleic acid according to claim 12, which is chosen from the group consisting of:
- the nucleic acid encoding an altered *M. tuberculosis* PZase polypeptide having the sequence of SEQ ID NO. 2 except for a-1 frameshift in the translational reading frame of the mRNA corresponding to amino acid position 96.
- the nucleic acid comprising a deletion of nucleotide 288 of the wild-type *pncA* gene, having the sequence of SEQ ID NO. 1,
- the nucleic acid encoding an altered *M. tuberculosis* PZase polypeptide having the amino acid sequence of SEQ ID NO. 2 except for a -1 frameshift in the translational reading &ame of the mRNA corresponding to amino acid position 54,
- the nucleic acid comprising a deletion of nucleotide 162 of the wild-type *pncA* gene having the sequence of SEQ ID NO-1,
- the nucleic acid encoding an altered *M. tuberculosis* PZase polypeptide having the sequence of SEQ ID NO. 2 except for a histidine residue at amino acid position 63,
- the nucleic acid comprising a change ofG to C at nucleotide 187 of the wild-type *pncA* gene having the sequence of SEQ ID NO. 1,
- the nucleic acid encoding an altered *M.* tuberculosis PZase polypeptide having the sequence of SEQ ID NO. 2 except for a serine residue at amino acid position 138,
- the nucleic acid comprising a change of T to A at nucleotide 412 of the wild-type *pncA* gene having the sequence of SEQ ID NO. 1,
- the nucleic acid encoding an altered *M.* tuberculosis PZase polypeptide having the sequence of SEQ ID NO. 2 except for a proline residue at amino acid position 141, and
- the nucleic acid comprising a change of A to C at nucleotide 422 of the wild-type *pncA* gene having the sequence of SEQ ID NO. 1.

16. A wild-type *pncA* nucleic acid according to claim 12 or 13 encoding a functional PZase for use as a medicament for treating a mammal infected with PZA-resistant mycobacteria.

17. Use of a wild-type *pncA* nucleic acid according to claims 12 or 13.encoding a functional PZase for the preparation of a medicament for treating a mammal infected with PZA-resistant mycobacteria.

18. The use of a *pncA* nucleic acid according to claim 17, wherein the mycobacteria are *M. bovis.*

19. The use of a *pncA* nucleic acid according to claim 17, wherein the mycobacteria are PZA-resistant *M*. *tuberculosis.*

20. The use of a wild-type *pncA* nucleic acid according to claim 17 in combination with a therapeutically effective amount ofPZA for the preparation of a medicament for treating a mammal infected with PZA-resistant mycobacteria.

21. The use according to claim 20 wherein the *pncA* nucleic acid and a therapeutically effective amount of PZA, in combination with a therapeutically effective amount of at least one other mycobacterial therapeutic agent are used for the preparation of a medicament for treating a mammal infected with PZA resistant mycobacteria.

22. The use according to claim 21, wherein the other mycobacterial therapeutic agent is selected from isoniazid and rifampicin.

23. The use according to any of claims 17 to 22, wherein the mammal is a human.

24. The use according to any of claims 17 to 23, wherein said wild-type *pnc*A nucleic acid is present in a mycobacteriophage.

25. A medicament comprising a mycobacteriophage as defined in claim 24.

26. A nucleic acid probe for identifying a PZA-resistant mycobacteriuni, wherein the probe comprises a nucleic acid that is complementary to a portion of an altered *pncA* gene of a PZA-resistant mycobacterium compared to the wild-type *M. tuberculosis pncA* gene encoding the wild-type PZase having the sequence of SEQ ID NO. 2, and the portion confers PZA-resistance.

27. The nucleic acid probe of claim 26, wherein the probe consists of 8 to 20 nucleotides.

28. The nucleic acid probe of claim 26, wherein the portion consists of 8 to 20 nucleotides.

29. Isolated oligonucleotide primer(s) for identifying PZA-resistant mycobacteria, wherein the primer hybridizes with a target polynucleotide sequence, said target polynucleotide sequence having the sequence selected from the group consisting of: and and sequences complementary thereto.

30. A kit useful for distinguishing *M. bovis* from *M. tuberculosis* or for identifying *M. bovis*, the kit comprising a container containing a pair of oligonucleotide primers for amplification of an altered portion of an *M*. *bovis pncA* gene compared to the wild-type *pncA M. tuberculosis* gene as identified in SEQ ID NO.1, said pair of primers flanking the altered portion of the *M. bovis pncA* gene and wherein the altered portion encodes a polypeptide comprising an aspartic acid residue at amino acid position 57 of the PZase polypeptide having the sequence of SEQ ID NO. 2.

31. The kit of claim 30, wherein the altered portion of the *pncA* gene comprises a change of C to G at nucleotide 169 of the wild-type *pncA* gene and wherein said primers are as defined in claim 3.

32. The kit of claim 30, wherein the primers amplify a 150-200 nucleotide portion of the *pncA* gene.

33. A kit useful for identifying a PZA-resistant mycobacterium, the kit comprising a container containing oligonucleotide primers for amplification of an altered portion of a *pnc*A gene according to claims 12 or 13.

34. A kit useful for identifying a PZA-resistant mycobacterium, the ldt comprising a nucleic acid probe of claim 26.

35. An isolated antibody that preferentially binds an PZase polypeptide which is altered compared to SEQ ID NO:2 and confers PZA resistance encoded by a nucleic acid according to any of claims 12 to 15.

36. The antibody of claim 35, wherein the antibody preferenti;illy binds an *M. bovis* PZase polypeptide encoded by a nucleic acid according to claim 14.

37. An altered PZase polypeptide encoded by a nucleic acid according to any of claims 12 to 15, or fragment thereof, that confers PZA resistance, or a conservative variant thereof.

38. The altered PZase polypeptide of claim 37, wherein me polypeptide is an *M*. *bovis* PZase polypeptide encoded by a nucleic acid according to claim 14.

39. A wild-type PZase polypeptide encoded by a nucleic acid according to claim 12 or 13 or a conservative variant thereof having the biological activity of SEQ ID NO 2.

## Patentansprüche

1. Ein Verfahren zur Differenzierung zwischen *M. tuberculosis* und *M. bovis* in einer Probe, wobei das Verfahren umfasst das Nachweisen eines veränderten *pncA*-Gens in der Probe, wobei das veränderte *pncA-*Gen für ein verändertes PZase-Polypeptid kodiert, das im Vergleich zu Wildtyp *M. tuberculosis* PZase, die die Sequenz von SEQ ID NR. 2 hat, einen Austausch eines Histidinrests gegen einen Aspartatrest an Aminosäureposition 57 umfasst, und das veränderte pncA-Gen darauf hin deutet, dass die Probe *M. bovis* umfasst.

2. Das Verfahren nach Anspruch 1, wobei der Austausch von Histidin nach Aspartat an Aminosäureposition 57 Ergebnis eines Austauschs von C nach G an Nukleotid 169 des veränderten *pncA*-Gens ist, im Vergleich zu Wildtyp *M. tuberculosis pncA*-Gen, das die Sequenz von SEQ ID NR. 1 hat.

3. Das Verfahren nach Anspruch 1 oder 2, wobei der Nachweis umfasst:
a) Amplifizieren einer Region der Nukleinsäure der Mykobakterien mittels eines Oligonukleotid-Primerpaars, das hybridisiert mit 5' und 3' flankierenden Polynukleotid-Sequenzen der Nukleinsäure, wobei die 5' und 3' flankierenden Polynukleotid-Zielsequenzen sind: und und komplementären Sequenzen dazu; und
b) Nachweisen der amplifizierten Region.

4. Das Verfahren nach Anspruch 3, wobei die Amplifikation mittels Polymerase-Kettenreaktion (PCR) erfolgt.

5. Das Verfahren nach Anspruch 1, wobei das veränderte *pncA*-Gen nachgewiesen wird mittels PCR-Einzelstrangkonformationspolymorphismus (PCR-SSCP), Einzelstrangkonformationspolymorphismus (SSCP), DNA-Sequenzierung, Nukleinsäure-hybridisierung, denaturierender Gradientengelelektrophorese, Ligase-vermitteltem Gennachweises oder RNase-Verdaus eines RNA/DNA-Duplex.

6. Ein Verfahren zur Identifizierung eines Pyrazinamid (PZA)-resistenten Mykobakteriums, wobei das Verfahren umfasst das Nachweisen eines veränderten *pncA*-Gens im Vergleich zu Wildtyp *M. tuberculosis pncA*-Gen in dem Mykobakterium, wobei das veränderte pncA-Gen PZA-Resistenz vermittelt und wobei das Wildtyp *M. tuberculosis pncA-*Gen kodiert für Wildtyp *M. tuberculosis* PZase, die die Sequenz von SEQ ID NR. 2 hat.

7. Das Verfahren nach Anspruch 6, wobei das veränderte pncA-Gen amplifiziert wird.

8. Das Verfahren nach Anspruch 7, wobei das veränderte *pncA*-Gen mittels Polymerase-Kettenreaktion (PCR) amplifiziert wird.

9. Das Verfahren nach Anspruch 8, wobei das veränderte *pncA*-Gen nachgewiesen wird mittels PCR-Einzelstrangkonformationspolymorphismus (PCR-SSCP), Einzelstrangkonformationspolymorphismus (SSCP), DNA-Sequenzierung, Nukleinsäurehybridisierung, denaturierender Gradientengelelektrophorese, Ligase-vermitteltem Gennachweises oder RNase-Verdaus eines RNA/DNA-Duplex.

10. Das Verfahren nach einem der Ansprüche 6 bis 9 wobei das Mykobakterium *M. tuberculosis* ist.

11. Das Verfahren nach einem der Ansprüche 6 bis 9 wobei die Veränderung im veränderten *pncA*-Gens gewählt wird von der Gruppe bestehend aus:
- die Veränderung in dem veränderten *pncA*-Gen kodiert für einen Aspartatrest an Aminosäureposition 57 im Vergleich zu Wildtyp PZase-Polypeptid von *M. tuberculosis,* das die Sequenz von SEQ ID NR.2 hat.
- die Veränderung im veränderten *pncA*-Gen umfasst einen Austausch von C nach G an Nukleotid 169 im Vergleich zum Wildtyp *pncA*-Gen, das die Sequenz von SEQ ID NR. hat,
- die Veränderung in dem veränderten *pncA*-Gen ist eine -1-Leserasterverschiebung in dem translationalen Leseraster auf der mRNA korrespondierend zu Aminosäureposition 96 von Wildtyp PZase-Polypeptid von *M. tuberculosis,* das die Sequenz von SEQ ID NR.2 hat,
- die Veränderung in dem veränderten *pncA*-Gen umfasst eine Deletion von Nukleotid 288 des Wildtyp *pncA*-Gens von *M. tuberculosis,* das die Sequenz von SEQ ID NR.1 hat,
- die Veränderung in dem veränderten *pncA*-Gen ist eine -1-Leserasterverschiebung in dem translationalen Leseraster auf der mRNA korrespondierend zu Aminosäureposition 54 von Wildtyp PZase-Polypeptid von *M. tuberculosis,* das die Sequenz von SEQ ID NR.2 hat,
- die Veränderung in dem veränderten *pncA*-Gen umfasst eine Deletion von Nukleotid 162 des Wildtyp *pncA*-Gens von *M. tuberculosis,* das die Sequenz von SEQ ID NR.1 hat,
- die Veränderung in dem veränderten *pncA*-Gen kodiert für einen Histidinrest an Aminosäuresposition 63 des Wildtyp PZase-Polypeptids von *M. tuberculosis,* das die Sequenz von SEQ ID NR.2 hat,
- die Veränderung in dem veränderten *pncA*-Gen umfasst einen Austausch von G nach C an Nukleotid 187 des Wildtyp *pncA*-Gens von *M. tuberculosis,* das die Sequenz von SEQ ID NR.1 hat,
- die Veränderung in dem veränderten *pncA*-Gen kodiert für einen Serinrest an Aminosäureposition 138 des Wildtyp PZase-Polypeptids von *M. tuberculosis,* das die Sequenz von SEQ ID NR.2 hat,
- die Veränderung in dem veränderten *pncA*-Gen umfasst einen Austausch von T nach A an Nukleotid 412 des Wildtyp *pncA*-Gens von *M. tuberculosis,* das die Sequenz von SEQ m NR.1 hat,
- die Veränderung in dem veränderten *pncA*-Gen kodiert für einen Prolinrest an Aminosäureposition 141 des Wildtyp PZase-Polypeptids von *M. tuberculosis,* das die Sequenz von SEQ ID NR.2 hat, und
- die Veränderung in dem veränderten *pncA*-Gen umfasst einen Austausch von A nach C an Nukleotid 422 des Wildtyp *pncA*-Gens von *M. tuberculosis,* das die Sequenz von SEQ ID NR.1 hat.

12. Eine isolierte Nukleinsäure, die für ein Wildtyp *M. tuberculosis* Pyrazinamidase (PZase)-Polypeptid kodiert, **gekennzeichnet durch** SEQ ID NR. 2, oder konservative Varianten davon, die die biologische Aktivität von SEQ ID NR. 2 haben, oder eine Nukleinsäure, die für ein verändertes *M. tuberculosis* PZase-Polypeptid im Vergleich zu Wildtyp *M. tuberculosis* PZase kodiert, das die SEQ ID NR.2 hat und PZA-Resistenz vermittelt.

13. Die Nukleinsäure nach Anspruch 12, wobei die Nukleinsäure die Nukleotidsequenz nach Figur 1 hat (SEQ ID NR.1) oder degenerierte Varianten davon, und für die Aminosäuresequenz von Figur 1 (SEQ ID NR.2) kodiert, die mit der Wildtyp *M. tuberculosis* PZase korrespondiert.

14. Eine isolierte Nukleinsäure, die für ein *M. bovis* PZase-Polypeptid kodiert, das einen Aspartatrest an Aminosäureposition 57 bezogen auf SEQ ID NR.2 hat.

15. Die isolierte Nukleinsäure nach Anspruch 12, die gewählt wird aus der Gruppe bestehend aus:
- die Nukleinsäure kodierend für ein verändertes *M. tuberculosis* PZase-Polypeptid, das die Sequenz von SEQ ID NR.2 hat, mit Ausnahme einer -1-Leserasterverschiebung in dem translationalen Leseraster der mRNA, korrespondierend zu Aminosäureposition 96,
- die Nukleinsäure umfassend eine Deletion von Nukleotid 288 des Wildtyp *pncA-*Gens, das die Sequenz von SEQ ID NR. 1 hat,
- die Nukleinsäure kodierend für ein verändertes *M. tuberculosis* PZase-Polypeptid, das die Aminosäuresequenz von SEQ ID NR. 2 hat, mit Ausnahme einer -1-Leserasterverschiebung in dem translationalen Leseraster der mRNA korrespondierend zu Aminosäureposition 54,
- die Nukleinsäure umfassend eine Deletion von Nukleotid 162 des Wildtyp *pncA-*Gens, das die Sequenz von SEQ ID NR. 1 hat,
- die Nukleinsäure kodierend für ein verändertes *M. tuberculosis* PZase-Polypeptid, das die Sequenz von SEQ ID NR. 2 hat, mit Ausnahme eines Histidinrests an Aminosäureposition 63,
- die Nukleinsäure umfassend einen Austausch von G nach C an Nukleotid 187 des Wildtyp *pncA*-Gens, das die Sequenz von SEQ ID NR.1 hat,
- die Nukleinsäure kodierend für ein verändertes *M. tuberculosis* PZase-Polypeptid, das die Sequenz von SEQ ID NR.2 hat, mit Ausnahme eines Serinrests an Aminosäureposition 138,
- die Nukleinsäure umfassend einen Austausch von T nach A an Nukleotid 412 des Wildtyp *pncA*-Gens, das die Sequenz von SEQ ID NR.1 hat,
- die Nukleinsäure kodierend für ein verändertes *M. tuberculosis* PZase-Polypeptid, das die Sequenz von SEQ ID NR.2 hat, mit Ausnahme eines Prolinrests an Aminosäureposition 141, und
- die Nukleinsäure umfassend ein Austausch von A nach C an Nukleotid 422 des Wildtyp *pncA*-Gens, das die Sequenz von SEQ ID NR. 1 hat.

16. Eine Wildtyp *pncA*-Nukleinsäure nach Anspruch 12 oder 13, die für eine funktionelle PZase kodiert, zur Verwendung als Medikament zur Behandlung eines Säugetiers, das mit PZA-resistenten Mykobakterien infiziert worden ist.

17. Verwendung einer Wildtyp *pncA*-Nukleinsäure nach Anspruch 12 oder 13, die für eine funktionelle PZase kodiert, zur Herstellung eines Medikaments zur Behandlung eines Säugetiers, das mit PZA-resistenten Mykobakterien infiziert ist.

18. Verwendung einer *pncA*-Nukleinsäure nach Anspruch 17, wobei die Mykobakterien *M. bovis* sind.

19. Verwendung einer *pncA*-Nukleinsäure nach Anspruch 17, wobei die Mykobakterien PZA-resistente *M. tuberculosis* sind.

20. Verwendung einer Wildtyp *pncA*-Nukleinsäure nach Anspruch 17 in Kombination mit einer therapeutisch effektiven Menge an PZA zur Herstellung eines Medikaments für die Behandlung eines Säugetiers, das mit PZA-resistenten Mykobakterien infiziert ist.

21. Verwendung nach Anspruch 20, wobei die *pncA*-Nukleinsäure und eine therapeutisch effektive Menge an PZA in Kombination mit einer therapeutisch effektiven Menge von mindestens einem weiteren mykobakteriell-therapeutischen Wirkstoff verwendet werden zur Herstellung eines Medikaments zur Behandlung eines Säugetiers, das mit PZA-resistenten Mykobakterien infiziert ist.

22. Verwendung nach Anspruch 21, wobei der weitere mykobakteriell-therapeutische Wirkstoff ausgewählt wird aus Isoniazid und Rifampicin.

23. Die Verwendung nach einem der Ansprüche 17 bis 22, wobei das Säugetier ein Mensch ist.

24. Die Verwendung nach einem der Ansprüche 17 bis 23, wobei die Wildtyp *pncA-*Nukleinsäure in einem Mykobakteriophagen vorliegt.

25. Ein Medikament umfassend einen Mykobakteriophagen wie in Anspruch 24 definiert.

26. Eine Nukleinsäuresonde zur Identifizierung eines PZA-resistenten Mykobakteriums, wobei die Sonde eine Nukleinsäure umfasst, die komplementär zu einem Teil eines veränderten *pncA*-Gens eines PZA-resistenten Mykobakteriums ist im Vergleich zum Wildtyp *M. tuberculosis pncA*-Gen kodierend die Wildtyp-PZase, die die Sequenz von SEQ ID NR. 2 hat, und einem Teil der PZA-Resistenz vermittelt.

27. Die Nukleinsäuresonde nach Anspruch 26, wobei die Sonde aus 8 bis 20 Nukleotiden besteht.

28. Die Nukleinsäuresonde nach Anspruch 26, wobei der Teil aus 8 bis 20 Nukleotiden besteht.

29. Isolierte(r) Oligonukleotidprimer zur Identifizierung PZA-resistenter Mykobakterien, wobei der Primer mit einer Zielpolynukleotidsequenz hybridisiert, wobei die Zielpolynukleotidsequenz eine Sequenz hat, die aus der Gruppe bestehend aus: und und dazu komplementären Sequenzen ausgewählt ist.

30. Ein Kit verwendbar zur Unterscheidung von *M. bovis* von *M. tuberculosis* oder zur Identifizierung von *M. bovis,* wobei der Kit umfasst einen Behälter beinhaltend ein Paar an Oligonukleotidprimem zur Amplifikation eines veränderten Teils an *M. bovis pncA-*Gen im Vergleich zu Wildtyp *pncA M. tuberculosis*-Gen wie dargestellt in SEQ ID NR. 1, wobei das Primerpaar den veränderte Teil des *M. bovis pncA*-Gens flankiert und wobei der veränderte Teil ein Polypeptid kodiert umfassend einen Aspartatrest an Aminosäureposition 57 des PZase-Polypeptids, das die Sequenz von SEQ ID NR. 2 hat.

31. Der Kit nach Anspruch 30, wobei der veränderte Teil des *pncA-*Gens einen Austausch von C nach G an Nukleotid 169 des Wildtyp *pncA*-Gens umfasst und wobei die Primer definiert sind wie in Anspruch 3.

32. Der Kit nach Anspruch 30, wobei die Primer einen 150-200 Nukleotideteil des *pncA-*Gens amplifizieren.

33. Ein Kit verwendbar zur Identifizierung eines PZA-resistenten Mykobakteriums, wobei der Kit umfasst einen Behälter beinhaltend Oligonukleotidprimer zur Amplifikation eines veränderten Teils des *pncA*-Gens nach Anspruch 12 oder 13.

34. Ein Kit verwendbar zur Identifizierung eines PZA-resistenten Mykobakteriums, wobei der Kit umfasst eine Nukleinsäuresonde nach Anspruch 26.

35. Ein isolierter Antikörper, der vorzugsweise an PZase-Polypeptid bindet, das verändert ist im Vergleich zu SEQ ID NR. 2 und das PZA-Resistenz vermittelt, kodiert durch eine Nukleinsäure nach einem der Ansprüche 12 bis 15.

36. Der Antikörper nach Anspruch 35, wobei der Antikörper vorzugsweise an *M. bovis* PZase-Polypeptid bindet, das von einer Nukleinsäure nach Anspruch 14 kodiert wird.

37. Ein verändertes PZase-Polypeptid kodiert durch eine Nukleinsäure nach einem der Ansprüche 12 bis 15, oder ein Fragment davon, das PZA-Resistenz vermittelt, oder eine konservative Variante davon.

38. Das veränderte PZase-Polypeptid nach Anspruch 37, wobei das Polypeptid ein *M. bovis* PZase-Polypeptid ist, das von einer Nukleinsäure nach Anspruch 14 kodiert wird.

39. Ein Wildtyp-PZase-Polypeptid kodiert durch eine Nukleinsäure nach Anspruch 12 oder 13 oder eine konservative Variante davon, die die biologische Aktivität von SEQ ID NR. 2 hat.

## Revendications

1. Procédé de différenciation entre *M. tuberculosis* et *M. bovis* dans un échantillon, le procédé comprenant la détection dans l'échantillon d'un gène *pncA* altéré, dans lequel le gène *pncA* altéré code un polypeptide PZase altéré comprenant une modification d'un résidu histidine en un résidu acide aspartique à la position d'acide aminé 57 comparé à la PZase de *M. tuberculosis* de type sauvage ayant la séquence de SEQ ID NO. 2 et le gène *pncA* altéré indique que l'échantillon contient *M. bovis.*

2. Procédé selon la revendication 1, dans lequel le remplacement de l'histidine par l'acide aspartique à la position d'acide aminé 57 résulte du remplacement de C par G au nucléotide 169 du gène *pncA* altéré comparé au gène *pncA* de *M. tuberculosis* de type sauvage ayant la séquence de SEQ ID NO. 1.

3. Procédé selon la revendication 1 ou 2, dans lequel la détection comprend :
a) l'amplification d'une région de l'acide nucléique de la mycobactérie au moyen d'une paire d'amorces oligonucléotidiques qui s'hybrident à des séquences cibles polynucléotidiques 5' et 3' flanquantes de l'acide nucléique, dans lesquelles lesdites séquences cibles polynucléotidiques 5' et 3' flanquantes sont: et et
des séquences complémentaires de celles-ci; et
b) la détection de la région amplifiée.

4. Procédé selon la revendication 3, dans lequel l'amplification est par réaction en chaîne à la polymérase (PCR).

5. Procédé selon la revendication 1, dans lequel le gène *pncA* altéré est détecté par polymorphisme de conformation PCR-double brin (PCR-SSCP), polymorphisme de conformation simple brin (SSCP), séquençage d'ADN, hybridation d'acide nucléique, électrophorèse sur gel en gradient de dénaturation, détection de gène par ligase, ou digestion par la RNase d'un duplex ARN/ADN.

6. Procédé d'identification d'une mycobactérie résistante au pyrazinamide (PZA), le procédé comprenant la détection d'un gène *pncA* altéré comparé au gène *pncA* de *M. tuberculosis* de type sauvage dans la mycobactérie, dans lequel le gène *pncA* altéré confère la résistance au PZA et dans lequel le gène *pncA* de *M. tuberculosis* de type sauvage code la PZase de *M. tuberculosis* de type sauvage ayant la séquence de SEQ ID NO.2.

7. Procédé selon la revendication 6, dans lequel le gène *pncA* altéré est amplifié.

8. Procédé selon la revendication 7, dans lequel le gène *pncA* altéré est amplifié par réaction en chaîne à la polymérase (PCR).

9. Procédé selon la revendication 8, dans lequel le gène *pncA* altéré est détecté par polymorphisme de conformation PCR-double brin (PCR-SSCP), polymorphisme de conformation simple brin (SSCP), séquençage d'ADN, hybridation d'acide nucléique, électrophorèse sur gel en gradient de dénaturation, détection de gène par ligase, ou digestion par la RNase d'un duplex ARN/ADN.

10. Procédé selon l'une quelconque des revendications 6 à 9, dans lequel la mycobactérie est *M. tuberculosis.*

11. Procédé selon l'une quelconque des revendications 6 à 9, dans lequel l'altération dans le gène *pncA* altéré est choisie dans le groupe consistant en:
- l'altération dans le gène *pncA* altéré code un résidu acide aspartique à la position d'acide aminé 57 comparé au polypeptide PZase de type sauvage de *M. tuberculosis* ayant la séquence de SEQ ID NO.2,
- l'altération dans le gène *pncA* altéré comprend un remplacement de C par G au nucléotide 169 comparé au gène *pncA* de type sauvage ayant la séquence de SEQ ID NO.1,
- l'altération dans le gène *pncA* altéré est un décalage de cadre de lecture -1 dans le cadre de lecture de traduction de l'ARNm correspondant à la position d'acide aminé 96 du polypeptide PZase de type sauvage de *M. tuberculosis* ayant la séquence de SEQ ID NO.2,
- l'altération dans le gène *pncA* altéré comprend une délétion du nucléotide 288 du gène *pncA* de type sauvage de *M. tuberculosis* ayant la séquence de SEQ ID NO.1,
- l'altération dans le gène *pncA* altéré est un décalage de cadre de lecture -1 dans le cadre de lecture de traduction de l'ARNm correspondant à la position d'acide aminé 54 du polypeptide PZase de type sauvage de *M. tuberculosis* ayant la séquence de SEQ ID NO.2,
- l'altération dans le gène *pncA* altéré comprend une délétion du nucléotide 162 du gène *pncA* de type sauvage de *M. tuberculosis* ayant la séquence de SEQ ID NO.1,
- l'altération dans le gène *pncA* altéré code un résidu histidine à la position d'acide aminé 63 du polypeptide PZase de type sauvage de *M. tuberculosis* ayant la séquence de SEQ ID NO.2,
- l'altération dans le gène *pncA* altéré comprend un remplacement de G par C au nucléotide 187 du gène *pncA* de type sauvage de *M. tuberculosis* ayant la séquence de SEQ ID NO.1,
- l'altération dans le gène *pncA* altéré code un résidu sérine à la position d'acide aminé 138 du polypeptide PZase de type sauvage de *M. tuberculosis* ayant la séquence de SEQ ID NO.2,
- l'altération dans le gène *pncA* altéré comprend un remplacement de T par A au nucléotide 412 du gène *pncA* de type sauvage de *M. tuberculosis* ayant la séquence de SEQ ID NO.1,
- l'altération dans le gène *pncA* altéré code un résidu proline à la position d'acide aminé 141 du polypeptide PZase de type sauvage de *M. tuberculosis* ayant la séquence de SEQ ID NO.2, et
- l'altération dans le gène *pncA* altéré comprend un remplacement de A par C au nucléotide 422 du gène *pncA* de type sauvage de *M. tuberculosis* ayant la séquence de SEQ ID NO.1.

12. Acide nucléique isolé codant un polypeptide pyrazinamidase (PZase) de *M. tuberculosis* de type sauvage **caractérisée par** SEQ ID NO.2, ou des variants conservatifs de celui-ci ayant l'activité biologique de SEQ ID NO. 2, ou un acide nucléique codant un polypeptide PZase altéré de *M. tuberculosis* comparé au PZase de type sauvage de *M. tuberculosis* ayant la séquence de SEQ ID NO.2 et conférant la résistance au PZA.

13. Acide nucléique de la revendication 12, dans lequel l'acide nucléique a la séquence nucléotidique de la Figure 1 (SEQ ID NO: 1), ou ses variants dégénérés, et code la séquence d'acides aminés de la Figure 1 (SEQ ID NO: 2) correspondant au type sauvage de la PZase de *M. tuberculosis.*

14. Acide nucléique isolé codant le polypeptide PZase de *M. bovis* ayant un résidu acide aspartique à la position d'acide aminé 57 par rapport à SEQ ID NO.2.

15. Acide nucléique isolé selon la revendication 12, qui est choisi parmi le groupe consistant en:
- l'acide nucléique codant un polypeptide PZase altéré de *M. tuberculosis* ayant la séquence de SEQ ID NO. 2 excepté pour un décalage de cadre de lecture -1 dans le cadre de lecture de traduction de l'ARNm correspondant à la position d'acide aminé 96,
- l'acide nucléique comprenant une délétion du nucléotide 288 du gène *pncA* de type sauvage, ayant la séquence de SEQ ID NO.1,
- l'acide nucléique codant un polypeptide PZase altéré de *M. tuberculosis* ayant la séquence d'acides aminés de SEQ ID NO. 2 excepté pour un décalage de cadre de lecture -1 dans le cadre de lecture de traduction de l'ARNm correspondant à la position d'acide aminé 54,
- l'acide nucléique comprenant une délétion du nucléotide 162 du gène *pncA* de type sauvage, ayant la séquence de SEQ ID NO.1,
- l'acide nucléique codant un polypeptide PZase altéré de *M. tuberculosis* ayant la séquence de SEQ ID NO. 2 excepté pour un résidu histidine à la position d'acide aminé 63,
- l'acide nucléique comprenant un remplacement de G par C au nucléotide 187 du gène *pncA* de type sauvage ayant la séquence de SEQ ID NO.1,
- l'acide nucléique codant un polypeptide PZase altéré de *M. tuberculosis* ayant la séquence de SEQ ID NO. 2 excepté pour un résidu sérine à la position d'acide aminé 138,
- l'acide nucléique comprenant un remplacement de T par A au nucléotide 412 du gène *pncA* de type sauvage ayant la séquence de SEQ ID NO.1,
- l'acide nucléique codant un polypeptide PZase altéré de *M. tuberculosis* ayant la séquence de SEQ ID NO. 2 excepté pour un résidu proline à la position d'acide aminé 141, et
- l'acide nucléique comprenant un remplacement de A par C au nucléotide 422 du gène *pncA* de type sauvage ayant la séquence de SEQ ID NO.1.

16. Acide nucléique *pncA* de type sauvage selon la revendication 12 ou 13 codant une PZase fonctionnelle pour l'utilisation comme un médicament pour traiter un mammifère infecté par des mycobactéries résistantes au PZA.

17. Utilisation d'un acide nucléique *pncA* de type sauvage selon les revendications 12 ou 13 codant une PZase fonctionnelle pour la préparation d'un médicament pour traiter un mammifère infecté par des mycobactéries résistantes au PZA.

18. Utilisation d'un acide nucléique *pncA* selon la revendication 17, dans laquelle les mycobactéries sont *M. bovis*.

19. Utilisation d'un acide nucléique *pncA* selon la revendication 17, dans laquelle les mycobactéries sont *M. tuberculosis* résistant à PZA.

20. Utilisation d'un acide nucléique *pncA* de type sauvage selon la revendication 17 en combinaison avec une quantité thérapeutiquement efficace de PZA pour la préparation d'un médicament pour traiter un mammifère infecté par des mycobactéries résistantes au PZA.

21. Utilisation selon la revendication 20, dans laquelle l'acide nucléique *pncA* et une quantité thérapeutiquement efficace de PZA, en combinaison avec une quantité thérapeutiquement efficace d'au moins un autre agent thérapeutique mycobactérien sont utilisés pour la préparation d'un médicament pour traiter un mammifère infecté par des mycobactéries résistantes au PZA.

22. Utilisation selon la revendication 21, dans laquelle l'autre agent thérapeutique mycobactérien est choisi parmi l'isoniazide et la rifampicine.

23. Utilisation selon l'une quelconque des revendications 17 à 22, dans laquelle le mammifère est un humain.

24. Utilisation selon l'une quelconque des revendications 17 à 23, dans laquelle ledit acide nucléique *pncA* de type sauvage est présent dans un mycobactériophage.

25. Médicament comprenant un mycobactériophage tel que défini dans la revendication 24.

26. Sonde d'acide nucléique pour identifier une mycobactérie résistante au PZA, dans laquelle la sonde comprend un acide nucléique qui est complémentaire d'une portion d'un gène *pncA* altéré d'une mycobactérie résistante au PZA comparé au gène *pncA* de *M. tuberculosis* de type sauvage codant la PZase de type sauvage ayant la séquence de SEQ ID NO.2, et la portion confère la résistance au PZA.

27. Sonde d'acide nucléique de la revendication 26, dans laquelle la sonde est constituée de 8 à 20 nucléotides.

28. Sonde d'acide nucléique selon la revendication 26, dans laquelle la portion est constituée de 8 à 20 nucléotides.

29. Amorce(s) oligonucléotidique(s) isolée(s) pour identifier des mycobactéries résistantes au PZA, dans laquelle (lesquelles) l'amorce s'hybride avec une séquence polynucléotidique cible, ladite séquence polynucléotidique cible ayant la séquence choisie dans le groupe consistant en: 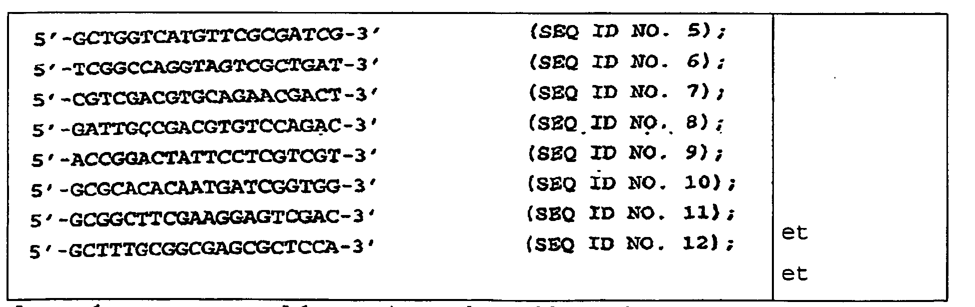 les séquences complémentaires de celles-ci.

30. Kit utile pour distinguer *M. Bovis* de *M. tuberculosis* ou pour identifier *M. bovis,* le kit comprenant un récipient contenant une paire d'amorces oligonucléotidiques pour l'amplification d'une portion altérée d'un gène *pncA* de *M*. *bovis* comparé au gène *pncA* de type sauvage de *M. tuberculosis* tel qu'identifié dans SEQ ID NO. 1, ladite paire d'amorces flanquant la portion altérée du gène *pncA* de *M. bovis* et dans lequel la portion altérée code un polypeptide comprenant un résidu acide aspartique à la position d'acide aminé 57 du polypeptide PZase ayant la séquence de SEQ ID NO. 2.

31. Kit de la revendication 30, dans lequel la portion altérée du gène *pncA* comprend un remplacement de C par G au nucléotide 169 du gène *pncA* de type sauvage et dans lequel lesdites amorces sont telles que définies dans la revendication 3.

32. Kit de la revendication 30, dans lequel les amorces amplifient une portion de 150-200 nucléotides du gène *pncA*.

33. Kit utile pour identifier une mycobactérie résistante au PZA, le kit comprenant un récipient contenant des amorces oligonucléotidique pour l'amplification d'une portion altérée d'un gène *pncA* selon les revendications 12 ou 13.

34. Kit utile pour identifier une mycobactérie résistante au PZA, le kit comprenant une sonde d'acide nucléique de la revendication 26.

35. Anticorps isolé qui lie préférentiellement un polypeptide PZase qui est altéré en comparaison à SEQ ID NO: 2 et confère la résistante au PZA codé par un acide nucléique selon l'une quelconque des revendications 12 à 15.

36. Anticorps de la revendication 35, dans lequel l'anticorps lie préférentiellement un polypeptide PZase de *M. bovis* codé par un acide nucléique selon la revendication 14.

37. Polypeptide PZase altéré codé par un acide nucléique selon l'une quelconque des revendications 12 à 15, ou un de ses fragments, qui confère la résistance au PZA, ou un de ses variants conservatifs.

38. Polypeptide PZase altéré de la revendication 37, dans lequel le polypeptide est un polypeptide PZase de *M. bovis* codé par un acide nucléique selon la revendication 14.

39. Polypeptide PZase de type sauvage codé par un acide nucléique selon la revendication 12 ou 13 ou un de ses variants conservatifs ayant l'activité biologique de SEQ ID NO 2.
